# EUROPEAN PATENT SPECIFICATION

(11) **EP 0 901 780 B1**
(45) Date of publication and mention of the grant of the patent: **22.01.2003**
(21) Application number: 95928014.0
(22) Date of filing: 10.08.1995
(51) Int. Cl.: A61F 13/15

(54) **ABSORBENT PRODUCT AND METHOD OF MANUFACTURING THE SAME**
ABSORBIERENDES PRODUKT UND VERFAHREN ZU SEINER HERSTELLUNG
ARTICLE ABSORBANT ET PROCEDE DE FABRICATION DE CET ARTICLE

(30) Priority: 24.08.1994 JP 19959994; 24.08.1994 JP 19960094; 24.08.1994 JP 19960194
(43) Date of publication of application: 17.03.1999
(73) Proprietor: Paragon Trade Brands, Inc., Norcross, Georgia 30092 (US)
(72) Inventor: SUZUKI, Migaku, Kanagawa 247 (JP); FUKUI, Hiroaki, Saitama 332 (JP)
(74) Representative: Dean, John Paul
(86) International application number: PCT/JP95/01601
(87) International publication number: WO 96/005788

(56) References cited:
- EP-A- 0 417 766
- EP-A- 0 446 867
- WO-A-94/28845
- JP-A- 4 028 365
- JP-A- 5 247 701
- JP-U- 3 107 919
- JP-U- 4 095 026
- US-A- 4 940 464

## Description

### Field of Art

The present invention relates to absorbent articles such as diapers for infants and adults, and sanitary articles, as well as a manufacturing method thereof.

### Background

Absorbent articles such as diapers for infants and adults can be roughly categorized as a tape-type and a pants-type. A tape-type diaper is composed of a rear waist portion and a front waist portion. When the tape-type diaper is used, the rear waist portion and the front waist portion are connected with detachable tapes. When the tape-type diaper is not in use, the rear waist portion and the front waist portion are kept in flat. On the other hand, a pants-type diaper has a waist hole portion and a pair of leg hole portions. In any type, a back sheet composed of a liquid impermeable material and a top sheet composed of a liquid permeable material disposed inside thereof are laid one on the other, and an absorbent member is disposed between the back sheet and the top sheet, thereby to form a main body. The main body has an elastic waist portion and elastic leg portions.

As disclosed in Japanese Patent Laid-Open Publication 3-82467, a conventional absorbent article is composed of a liquid permeable top sheet, a liquid impermeable back sheet, and an absorbent. The absorbent member is disposed between the liquid permeable top sheet and the liquid impermeable back sheet. The top sheet and the back sheet are heat-bonded. In addition, to prevent a body exudate from leaking out, a pair of elastic leg gathers are disposed along the peripheries of the leg holes.

In conventional absorbent articles, it is known that the leakage protective function which is the most important function thereof cannot satisfy various requirements corresponding to applications, conditions, and so forth. For example, the leakage protective effect of disposable diapers depends on the fit between the leg holes and the legs of the wearer. Thus, it is considered that, as the fit improves, the probability of leakage decreases. Consequently, as the elastic force of the gathers disposed along the leg holes or leg gathers increases, the degree of the leakage decreases. However, when the elastic force is too large, the leg gathers are caught in the legs of the wearer. Therefore, when the wearer wears the absorbent material for a long time, the wearer not only feels a pain, but suffers a skin inflammation such as an eruption.

A known absorbent article representing the closest prior art is described in EP-A-0446867.

An aim of the present invention is to provide an absorbent article which solves the above-described problems involved in the conventional absorbent articles, and has a very excellent leakage protective effect in such a manner that the wearer is free of an excessive pressure.

Another aim of the present invention is to provide a manufacturing method of the above-described absorbent article.

### Description of the Invention

The present invention provides an absorbent article comprising a main body comprising a front portion, a rear portion, a waist hole and a pair of leg holes disposed between the front portion and the rear portion, a respective elastic leakage protective member mounted at a site corresponding to each leg hole of the main body to interconnect edge portions of the front and rear portions so that at least part of the respective leg hole is closed, characterised in that each of the leakage protective members has an area accounting for at least two thirds of the area of the respective leg hole.

As with the conventional absorbent articles, in the absorbent article according to the present invention, a main body is composed of a liquid impermeable outer sheet, a liquid permeable inner sheet, and an absorbent member disposed therebetween. A front portion which covers the waist front portion and a rear portion which covers the waist rear portion may be integrally formed with an outer sheet, an inner sheet, and an absorbent. Alternatively, a front portion composed of an outer sheet, an inner sheet, and an absorbent member, and a rear portion composed of an outer sheet, an inner sheet, and an absorbent member may be separately prepared. The rear portion and the front portion are connected at the crotch portions thereof.

The absorbent member may be disposed between the outer sheet and the inner sheet. However, an absorbent member sandwiched with a liquid permeable sheet and a liquid impermeable sheet may be disposed inside the inner sheet.

In this specification, the term "leakage protective member" means a sheet member which is disposed on both sides of the above-described main body, and composed of an elastic sheet forming at least part of the periphery of a leg hole, and prevents body exudate from leaking. The elastic leakage protective member may have a variety of forms, such as a standing cuff disposed at each side of the main body, and a vessel as a pocket for holding body exudate.

The area of each leg hole is defined so that each leg of a wearer smoothly passes, and the leg hole properly fits the leg. Experimental results show that, due to elasticity, the elastic leakage protective member according to the present invention properly satisfies the above-described conditions in the case that the elastic leakage protective member closes more than 2/3 of the opening of the leg hole.

The elastic leakage protective member may have a slit which has a predetermined length, and which extends from a portion which constitutes part of the periphery of the leg hole. The slit allows a leakage protective member to have a large area, and to secure a large area of the opening.

Thus, urine and excrement are sealed by an elastic leakage protective member which is disposed at a lower crotch portion and follows the motion of the wearer. The elastic leakage protective member is secured to the crotch portion with the upper crotch portion of the main body.

In another structure of the present invention, the elastic leakage protective member may have a slit which has a predetermined length and extends from the upper edge portion. With the slit, the elastic leakage protective member can cover most portions of the leg holes and thereby the leg. Thus, a satisfactory large opening area can be obtained.

In the structure of which the entire periphery of the crotch portion is covered by the elastic leakage protective member, the absorbent article can freely follow the motion of the wearer (namely, the wearer can freely run and walk). This structure is very effective for a tape-less type diaper so-called pans-type diaper.

The present invention also provides an absorbent article comprising a main body comprising a front portion, a rear portion, a waist hole, and a pair of leg holes disposed between the front portion and the rear portion, a respective elastic leakage protective member mounted at a site corresponding to each leg hole of the main body to interconnect edge portions of the front and rear portions so that at least part of the respective leg hole is closed, characterised in that each of the leakage protective members has a width which is widest at a part lying at the centre of the thigh portion of the main body, and which becomes narrower at the waist hole side than that.

The present invention further provides a method of manufacturing an absorbent article having a main body comprising a front portion and a rear portion, the method comprising the steps of: conveying a first continuous elongated strip-like assembly prepared for forming the front portion, in its longitudinal direction; supplying continuously to both edges of the first assembly a pair of sheet materials each forming a continuous strip-like member folded into two layers at its centre as viewed in its width direction and the width direction of the first continuous elongated strip assembly, and having openings formed at a predetermined interval; superimposing a second continuous strip-like assembly, which serves as the rear portion, onto the first assembly with the sheet-shaped material superimposed thereon while conveying in its longitudinal direction; joining the first assembly, the second assembly and the sheet material at a predetermined position for integration; and cutting off the integrated first assembly, the second assembly and the sheet-shaped material at a predetermined interval to obtain a discrete absorbent article, in which each of the pair of sheet materials interconnects edge portions of the first assembly and the second assembly such that the openings in the sheet materials form leg holes.

In the absorbent article according to the present invention with such a structure, each of the elastic leakage protective members fits the leg of a wearer with a large area, thereby improving comfortableness and excellent leakage protective effect.

According to another aspect of the present invention, a second portion of the elastic leakage protective member has a size or shape so that it functions as a separator for separating the crotch region of the main body from the other regions.

In this case, since the crotch portion which holds body exudate is separated from the other regions, even if the amount of body exudate exceeds the absorbing speed of the absorbent, the elastic leakage protective member holds and gradually absorbs it. Thus, the absorbent article has an excellent leakage protective effect.

When the second portion of the elastic leakage protective member is disposed before and behind the crotch portion of a wearer or therearound, the inside of the elastic leakage protective member forms a pocket separated from the outside, thereby further improving the separator function.

The present invention further provides a method of manufacturing an absorbent article having front and rear portions interconnected at side edge portions and the crotch portions thereof, leakage protective members made of an elastic sheet material interconnecting the facing side edge portions of the front and rear portions over the entire length extending from the side edge portion of the waist up to the side edge portion of the crotch portion, each leakage protective member being divided into an upper zone and a lower zone by means of an opening formed at a predetermined position of an area extending from its upper edge up to its lower edge, the method comprising the steps of: superimposing continuous strip-like top and back sheets to prepare a first assembly formed with an absorbent member therebetween at a proper interval, the first assembly serving as the front portion; superimposing continuous strip-like top and back sheets to prepare a second assembly formed with an absorbent member sandwiched therebetween at a proper interval, the second assembly serving as the rear portion; continuously supplying the first and second assemblies at a predetermined point of their transfer path at a speed synchronised so as to be superimposed at such a timing at which each absorbent member faces each other; folding a continuous sheet material, which serves as the leakage protective member, into two at the central portion as viewed in its width direction and forming the opening at a predetermined interval to form a member adapted for the leakage protective member continuously supplying a sub-assembly between the first and second assemblies, folded into two at a point lying ahead of the point; bonding the first and second assemblies and the member adapted for the leakage protective member at a predetermined binding portion; and cutting the first and second assemblies along a predetermined cutting line to separate into individual absorbent articles.

The advantage of this method is in that standing cuffs having sufficient leakage protective effect can be disposed on the main body.

The present invention still further provides an absorbent article having a front portion and a rear portion interconnected together at side edge portions thereof, the article comprising: leakage protective members of a resilient sheet material interconnecting each facing side edge portions of the front portion and the rear portion over the entire length extending from the side edge portion of the waist up to the side edge portion of the crotch portion; and each leakage protective member being divided into an upper zone and a lower zone by means of an opening formed at a predetermined position of the area extending from its upper edge up to its lower edge; whereby the opening of each leakage protective member functions as a leg hole while one wears this article, with the wearer's leg inserted into the leg hole, the upper zone is developed outwardly so as to come in contact with the wearer's waist portion, and the lower zone is developed inwardly so as to come in contact with the inner side of the wearer's leg.

The basic concept of the present invention is to fit the absorbent article to the waist portion and the crotch portion of a wearer with a single elastic leakage protective member, unlike with waste gather and leg gathers of the conventional absorbent articles.

The waist portion of the conventional diaper should have a function for allowing the waist of the wearer to fit the diaper. However, no special attention is paid for leakage. Thus, the entire periphery of the waist portion which covers the waist of the wearer does not need elasticity. On the other hand, the joints of the femoral portions (hereinafter referred to as femoral base portions) are subject to the motion of the wearer. In addition, the femoral base portions should have excellent leakage protective effect. Thus, the absorbent article should have a structure of which it fits the entire peripheries of the femoral base portions. However, as described above, when the elasticity of the portions which fit the femoral base portions is increased, various problems take place.

According to the present invention, a single elastic leakage protective member is sectioned as an upper zone and a lower zone at an opening portion. In addition, these zones are structure so that they share functions of which the upper zone fits the waist portion and the outside of the femoral base portions, and the lower zone fits the inside of the femoral base portions. Thus, the waist portion can have necessary and satisfactory fitness. The lower edge of the upper zone inwardly covers the femoral base portions. The upper edge of the lower zone outwardly covers the femoral base portions. Thus, the absorbent article provides both excellent fitness and leakage protective effect.

Such functional sharing allows shrinking force due to elasticity of the elastic leakage protective members to disperse. Thus, the absorbent article smoothly fits the body of the wearer, thereby providing satisfactory fitness and leakage protective effect. Consequently, since the wearer is not subject to excessively pressure, the above-described problems do not take place. In addition, since large elasticity is not required, the amount of elastic material can be reduced. Thus, the cost of the material can be reduced and the manufacturing process can be simplified.

Moreover, when the absorbent article fits the femoral base portions, force hardly interferes between the upper zone and the lower zone. Thus, since the degree of freedom of designing the structure of an absorbent article becomes large, products with features corresponding to various applications can be easily developed.

Another advantage of the present invention is in that a front portion and a rear portion which have a relatively stable strip structure are connected with a flexible and thin elastic leakage protective member which does not have a layered side seal connection portions. In addition, since the elastic leakage protective member is internally folded, the thickness thereof is as small as the total thickness of the front portion and the rear portion. Thus, after the products are manufactured, they can be easily packaged and transported. In addition, the users can easily store and handle the products.

The present invention is also a method of manufacturing an absorbent article having front and rear portions interconnected at both side edge portions and the crotch portions thereof, leakage protective members made of an elastic sheet material interconnecting the facing side edge portions of the front and rear portions over the entire length extending from the side edge portion of the waist up to the side edge portion of the crotch portion, each leakage protective member being divided into the upper zone and the lower zone by means of an opening formed at a predetermined position of an area extending from its upper edge up to its lower edge, comprising superimposing continuous strip-like top sheet and back sheet to prepare a first assembly formed with an absorbent member therebetween at a proper interval, the first assembly serving as the front portion, superimposing continuous strip-like top sheet and back sheet to prepare a second assembly formed with an absorbent member sandwiched therebetween at a proper interval, the second assembly serving as the rear portion, continuously supplying the first and second assemblies at a predetermined point P1 of their transfer path at a speed synchronized so as to be superimposed at such a timing at which each absorbent member faces each other, folding a continuous sheet material, which serves as the leakage protective member, into two at the central portion as viewed in its width direction and forming the opening at a predetermined interval to form a member adapted for leakage protective member, continuously supplying the subassembly between the first and second assemblies, folded into two at a point P2 lying ahead of the point P1, bonding the first and second assemblies and the member adapted for leakage protective member at a predetermined bonding portion, and cutting the first and second assemblies along a predetermined cutting line to separate into individual absorbent articles.

The advantage of this method is in that elastic leakage protective members which have both functions of waist bands and standing cuffs with sufficient leakage protective effect can be disposed in a small number of steps. In addition, since the sheet material of the elastic leakage protective members is inwardly two-folded at the center portion in the width direction before it is supplied between the first and second assemblies up to the last step, each product is cut and .........

The advantage of this method is in that elastic leakage protective members which have both functions of waist bands and standing cuffs with sufficient leakage protective effect can be disposed in a small number of steps. In addition, since the sheet material of the elastic leakage protective members is inwardly two-folded at the center portion in the width direction before it is supplied between the first and second assemblies up to the last step, each product is cut and separated in the minimum direction.

In the manufacturing process of the conventional "pants-type absorbent article", the elastic leakage protective member is disposed and folded in the following three steps. At a first step, both side portions of the main body and the elastic leakage protective members are connected. At a second step, the front portion and the rear portion are two-folded so that their edges thereof are matched. At a third step, each product is cut and separated and the connected elastic leakage protective members being connected are inwardly folded.

When such three steps are used, depending on the alignment and connecting condition, the failure occurrence ratio increases and the manufacturing speed decreases. Thus, the manufacturing efficiency is adversely affected. However, according to the present invention, such three processes can be omitted, the products can be easily transported and packaged. In addition, the amount of packaging material can be reduced.

### Brief Description of Drawings

Fig. 1 is a perspective view showing an absorbent article according to a first aspect of the present invention in the state that a wearer wears the absorbent article;
Fig. 2 is an exploded perspective view showing an absorbent article shown in Fig. 1;
Fig. 3 is a perspective view showing an elastic leakage protective member for use with an absorbent article according to another aspect of the present invention;
Figs. 4A and 4B are plan views showing elastic leakage protective members for use with an absorbent article according to another aspect of the present invention;
Fig. 5 is a perspective view showing an absorbent article according to a second aspect of the present invention;
Fig. 6 is a schematic diagram for explaining an elastic leakage protective member for use with the absorbent article shown in Fig. 5;
Fig. 7 is a schematic diagram for explaining an elastic leakage protective member for use with another absorbent article similar to the absorbent article shown in Fig. 5;
Fig. 8 is a perspective view showing an absorbent article according to a third aspect of the present invention in the state that a wearer wears the absorbent article;
Figs. 9A and 9B are plan views showing other elastic leakage protective members for uses with the absorbent article shown in Fig. 8;
Figs. 10A, 10B, and 10C are plan views showing other elastic leakage protective members for use with the absorbent article shown in Fig. 8;
Figs. 11A and 11B are plan views showing other elastic leakage protective members for use with the absorbent article shown in Fig. 8;
Fig. 12 is a perspective view for explaining manufacturing steps of the absorbent article shown in Fig. 7;
Fig. 13 is a perspective view showing an absorbent article according to a forth aspect of the present invention;
Fig. 14 is an exploded perspective view showing the absorbent article shown in Fig. 13;
Figs. 15A to 15E are plan views for explaining steps for manufacturing an elastic leakage protective member for use with the absorbent article shown in Figs. 13 and 14;
Fig. 16 is a perspective view showing a step for placing the elastic leakage protective member shown in Fig. 15 to a main body;
Fig. 17 is an exploded perspective view showing an absorbent article according to a fifth aspect of the present invention;
Fig. 18 is an exploded perspective view showing a absorbent article according to a sixth aspect of the present invention;
Fig. 19 is an exploded perspective view showing an absorbent article according to a seventh aspect of the present invention;
Fig. 20A is a front view showing a sheet member structuring an elastic leakage protective member for use with the absorbent article shown in Fig. 19;
Fig. 20B is a rear view showing the sheet member shown in Fig. 20A;
Fig. 21 is a partial exploded view showing an absorbent article which is not used according to an eighth aspect of the present invention;
Fig. 22A is a partial sectional view taken along line A shown in Fig. 21;
Fig. 22B is a partial sectional view taken along line B shown in Fig. 21;
Fig. 23 is a partial exploded view showing the state that an upper zone of an elastic leakage protective member of the absorbent article shown in Fig. 21 is pulled out before the absorbent article is used;
Fig. 24 is a schematic diagram for explaining the absorbent article shown in Fig. 21 in the state that a wearer wears the absorbent article;
Fig. 25 is a schematic diagram for explaining the absorbent article shown in Fig. 21 in the state that a wearer wears the absorbent article;
Fig. 26 is a perspective view showing a part of an elastic leakage protective member for use with the absorbent article shown in Fig. 21;
Fig. 27 is an exploded plan view showing a part of an elastic leakage protective member for use with the absorbent article shown in Fig. 21;
Fig. 28 is a schematic diagram for explaining the area of an opening formed in an elastic leakage protective member;
Fig. 29 is an exploded plan view showing another elastic leakage protective member for use with an absorbent article according to the present invention;
Fig. 30 is an exploded plan view showing another elastic leakage protective member for use with an absorbent article according to the present invention;
Fig. 31 is a schematic diagram for explaining a longitudinal manufacturing step of an absorbent article according to the present invention; and
Fig. 32 is a schematic diagram for explaining a lateral manufacturing step of an absorbent article according to the present invention.

### Best Modes for Carrying out the Invention

The present invention will be described with reference to the accompanying drawings.

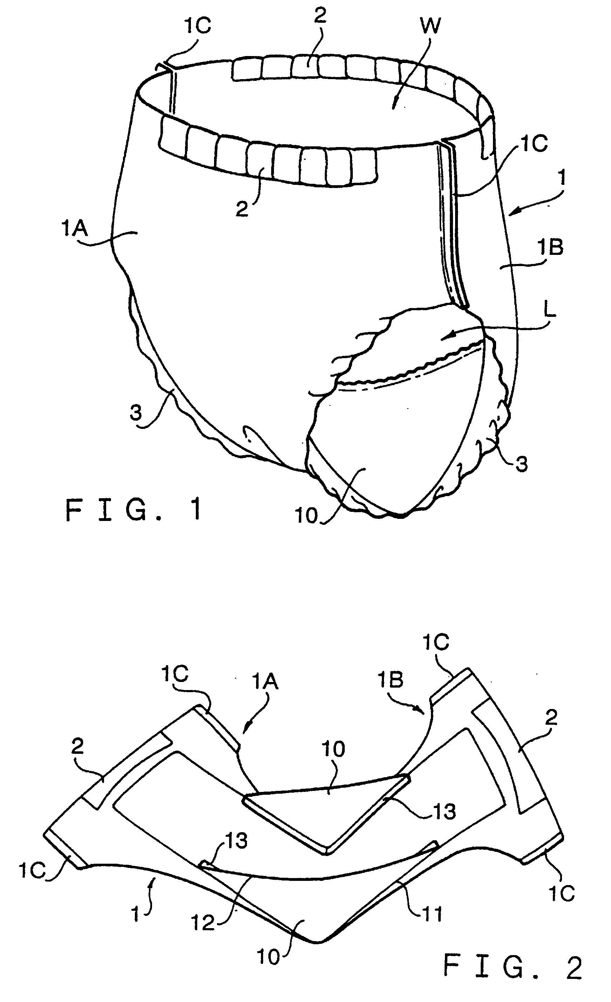

Fig. 1 is a perspective view showing a tape-less type diaper absorbent article according to a first aspect of the present invention in the state that a wearer wears the absorbent article. In the state that a wearer wears the absorbent article, one waist hole W and two leg holes L are formed on a main body 1. An elastic leakage protective member 10 is disposed at a lower portion of each of the leg holes L so that it covers at least part of the lower portion (in this embodiment, around 2/3 of the lower portion). When necessary, a waist gather 2 and a leg gather 3 are disposed along the waist hole W and each of the leg holes L so as to provide them with elasticity.

Fig. 2 is an exploded view of Fig. 1. In Fig. 2, the main body 1 is of conventional pants-type having a liquid permeable top sheet, a liquid impermeable back sheet, and a absorbent member disposed therebetween. The main body 1 has a front portion 1A and a rear portion 1B. The front portion 1A covers the abdomen portion of the wearer. The rear portion 1B covers the hips portion. The main body 1 is formed in a rectangular shape of which the longitudinal center portion is narrowed. Each edge portion of the front portion 1A and each edge portion of the rear portion 1B are connected with respective connection portions 1C. The present invention can be applied for a tape-type absorbent article. In this case, tapes which detachably connect the front portion are disposed at both edge portions of the rear portion of the main body 1. Since the structure of the main body 1 is basically the same as the structure of conventional absorbent articles, detail description of the main body 1 is omitted. In addition, for simplicity, Fig. 2 does not show the leg gathers 3.

In addition, a pair of elastic leakage protective members 10 are disposed on both sides of the center portion (namely, the crotch portion) of the main body 10. Each of the elastic leakage protective members 10 is formed in a nearly isosceles triangle shape surrounded by a V-shaped or U-shaped lower edge 11 and a straight or arc-shaped upper edge 12. A strip-shaped connection region 13 of which the almost inside of the lower edge 11 is folded is connected to the inner front surface of the crotch portion of the main body 1 with a proper bonding agent such as an hot-melt type bonding agent.

The material of the elastic leakage protective member 10 is preferably a sheet-shaped material which has comfortable touch, proper elasticity, high flexibility, and good leakage resistance. Examples of the sheet-shaped material are non-woven fabric with proper flexibility and elasticity and composite material of non-woven fabric and elastic substance. An example of non-woven fabric is elastic non-woven fabric which is manufactured by alternating fabric of polyester, polypropylene, or the like with a card web which contains conjugated fibers in a water stream and then heat-shrinking the resultant substance. Preferable examples of the elastic composite substance are a composite substance of non-woven fabric and elastic film, a composite substance of non-woven fabric and elastic melt-brown, and a composite substance of non-woven fabric and net-shaped elastic. Although an elastic film may be used, it directly touches the skin of the wearer. Thus, the elastic film should be used along with for example non-woven fabric.

A more preferable example of the elastic leakage protective member is a material with air permeability. When necessary, a plurality of small air pores which are free from liquid are formed at the upper portion of each of the elastic leakage protective members so as to effectively prevent stuffiness.

In addition, the elastic leakage protective members 10 which cover part of the respective leg holes L do not prevent the legs of the wearer from passing due to the elasticity thereof. In the state that the wearer wears the-absorbent article, the elastic leakage protective members 10 fit the legs along with the leg gathers 3 and secure the absorbent article at a predetermined position.

In the embodiment shown in Figs. 1 and 2, the strip-shaped connection region 13 is formed by inwardly folding part of each of the elastic leakage protective members 10. Occasionally, however, the connection region 13 may be formed by outwardly folding it.

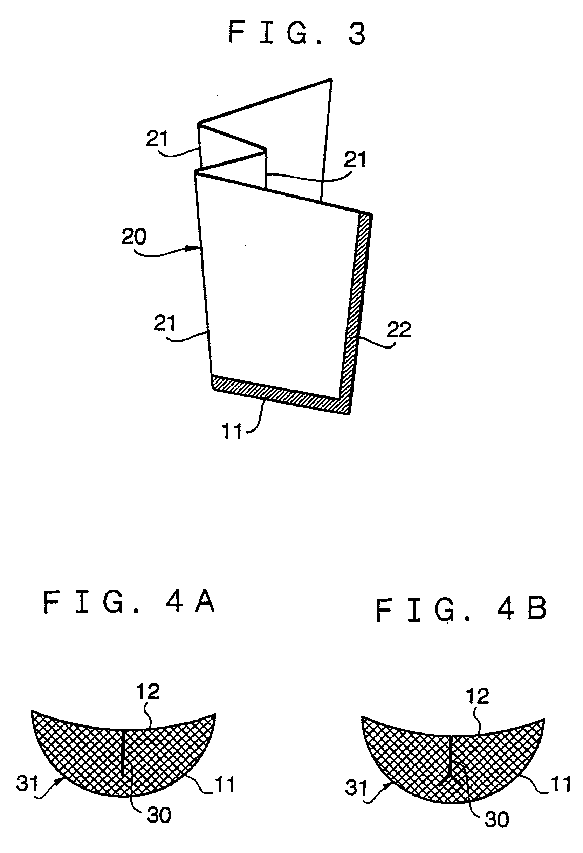

Alternatively, as shown in Fig. 3, leakage protective members 20 of which a nearly rectangular or trapezoidal sheet-shaped material is folded in a zigzag pattern along three folding lines 21 which are almost in parallel may be used. The elastic leakage protective members 20 are connected to predetermined positions of the main body 1 with a bonding agent 22 such as a hot-melt type bonding agent in such a manner that the bonding agent 22 is applied in the full length of the lower edge of each of the elastic leakage protective members 20 and the side edge of a segment disposed at both ends thereof. Since the length of the folded elastic leakage protective members 20 is longer than the length of the flat elastic leakage protective members 10 shown in Figs. 1 and 2, the degree of freedom of the expansion in the direction of which the front portion is separated from the rear portion of the former is higher than that of the latter.

Figs. 4A and 4B show elastic leakage protective members according to another aspect of the present invention.

In these embodiments, a slit 31 with a predetermined length is formed in a leakage protective member 30 composed of an elastic sheet-shaped material. The slit 31 extends from the center position of an upper edge 12 of the leakage protective member 30 to a lower edge thereof. In the embodiment shown in Fig. 4A, the slit 31 is a straight line. In the embodiment shown in Fig. 4B, the slit 31 branches to two ways at the lower position. In any case, in the state that the elastic leakage protective members 30 are disposed in the main body 1, when a leg of the wearer passes the leg hole L, the slit 31 allows the upper portion of the elastic leakage protective member 30 to largely open. Thus, even if the elastic leakage protective member 30 has a size of which it covers the most of the leg hole L, the elastic leakage protective member 30 does not prevent the leg of the wearer from passing the leg hole L. In addition, since the elastic leakage protective member 30 touches the leg for the most of the periphery of the leg hole L, the fitness of the absorbent article improves.

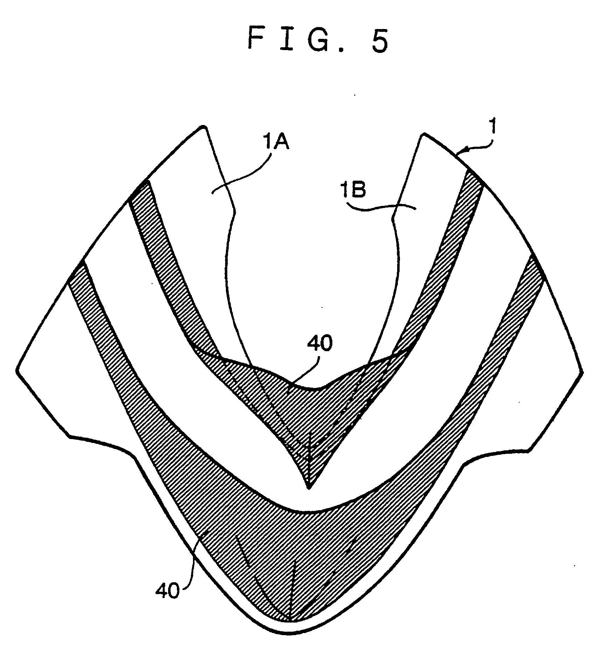

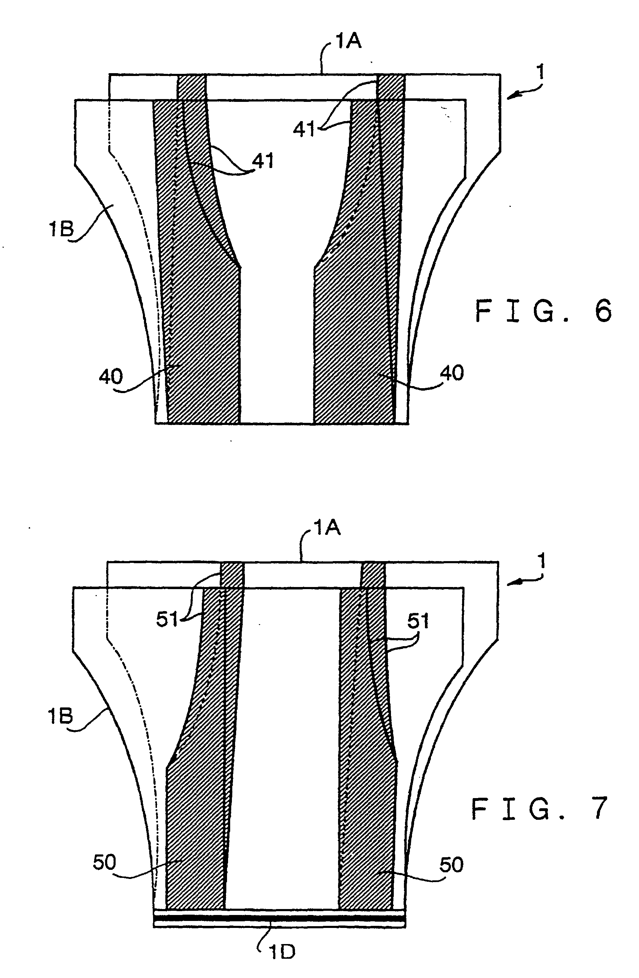

Figs. 5 and 6 show an absorbent article according to a second aspect of the present invention. In this case, each of elastic leakage protective members 40 disposed in a main body 1 is composed of an elongated strip-shaped elastic material. Each of the elastic leakage protective members 40 has the largest width at the center position of the crotch portion of the main body 1. In other words, the width of the elastic leakage protective member 40 on the waist hole side is smaller than that on the crotch portion side. As a means for varying the width of the elastic leakage protective member 40, the elastic leakage protective member 40 is cut out on both the edge sides thereof for a predetermined length. In the embodiment shown in Figs. 5 and 6, the elastic leakage protective members 40 are connected at an outer edge portion of the main body 1. However, with the elastic leakage protective members 40, as shown in Fig. 5, in the state that a wearer wears the absorbent article, when the leg holes are enlarged, edge portions which are not bonded to the main body 1 are raised and form cuffs which cover at least part of the leg holes as with the above-described embodiment.

Fig. 5 shows that one elastic leakage protective member is disposed to the main body 1 with the inner edge portion thereof and the other elastic leakage protective member is disposed to the main body 1 with the outer edge portion thereof. For simplicity, the two elastic leakage protective members disposed to the main body are shown with one drawing. Thus, it does not mean that the left portion and right portion of the elastic leakage protective members differ from each other.

Alternatively, as shown in Fig. 7, elastic leakage protective members 50 may be formed in such a manner that two-folded sheet materials are disposed to the main body 1 and that each open side thereof faces inside. In this case, the rise portions (which cover part of the leg holes in state that a wearer wears the absorbent article) of the elastic leakage protective members 50 are placed outside the connection line of the main body 1.

In the embodiment shown in Fig. 7, the main body 1 has a front portion 1A and a rear portion 1B as independent members which are mutually connected at connection portions 1D of the side edges of the crotch portions thereof. The elastic leakage protective members 50 are disposed to the main body 1 in such a manner that cut-out portions 51 which are the same as those shown in Figs. 5 and 6 face outwardly. In this case, when the wearer wears the absorbent article, the elastic leakage protective members 50 rise so that they fit the body of the wearer and prevent body exudate from leaking.

The main body 1 shown in Fig. 7 is suitable for a manufacturing method in which a first assembly for front portions of which absorbent members are disposed between sheet materials for back sheets and top sheet at predetermined intervals and a second assembly for rear portions which is similar to the first assembly are conveyed in parallel and an elastic sheet material is continuously supplied therebetween. The first assembly, the second assembly, and absorbent article material are connected in position. Each front portion 1A and each rear portion 1B are connected at a connection portion 1D.

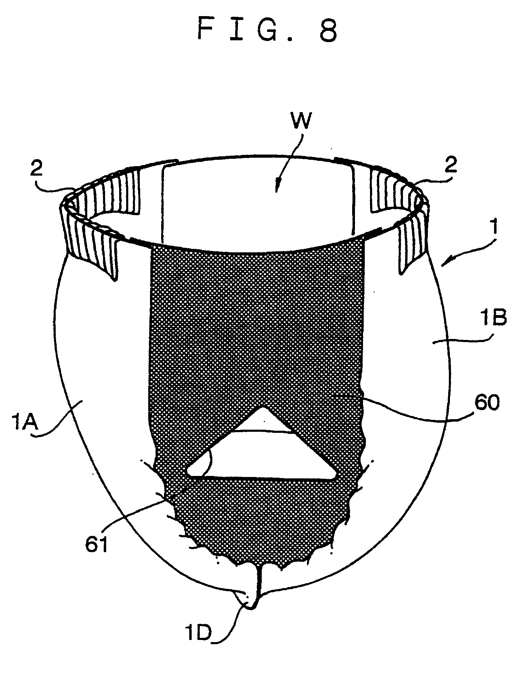

Fig. 8 shows an absorbent article according to a third aspect of the present invention. In this embodiment, a pair of elastic leakage protective members 60 are disposed to a main body 1 in such a manner that the elastic leakage protective members 60 connect both edge portions of a front portion 1A of the main body 1 and a rear portion 1B thereof. Thus, the upper edge portions of the front portion 1A, the rear portion 1B, and the elastic leakage protective members 60 are connected in a ring shape and thereby a waist hole W is formed. Each of the elastic leakage protective members 60 has an opening 61 formed in a nearly isosceles triangle shape. The opening 61 becomes a leg hole.

In this structure, each of the elastic leakage protective members 60 functions as a cuff which fits the periphery of a leg of the wearer and prevents body exudate from leaking. In addition, the elastic leakage protective member 60 functions as a leakage protective band which connects the front portion and the rear portion on their side positions. Thus, with such a structure, the absorbent article provides more higher fitness.

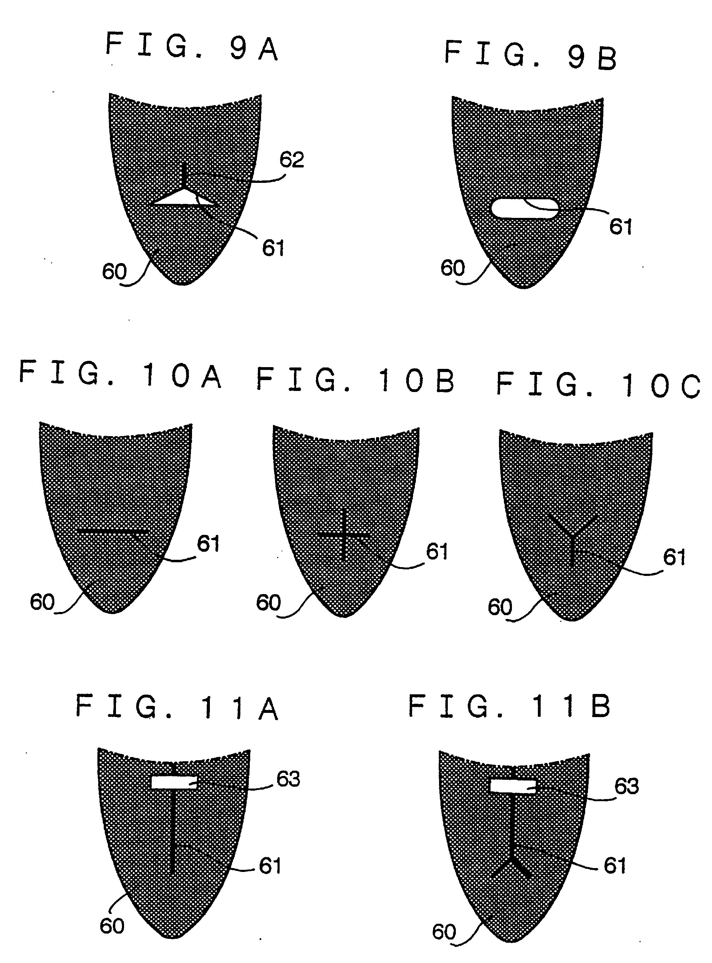

In the case that the opening 61 of the elastic leakage protective member 60 is formed in a triangular shape as shown in Fig. 8, when necessary, a slit 62 which upwardly extends from the vertex of the opening 61 may be formed as shown in Fig. 9A. The slit 62 also functions as a tearing edge for tearing the elastic leakage protective member 60 from the opening 61 when the wearer removes the absorbent article. When the strength necessary for tearing the material of the elastic leakage protective member 60 is large, a perforation which extends from the vertex of the opening 61 to the upper edge of the elastic leakage protective member 60 may be formed.

When the height of the triangular opening 61 (namely, the angle of the base and a side which contacts the base) is properly selected, the sum of the three sides of the triangle (namely, the size of the opening as the leg hole) can be selected from a wide range.

Alternatively, as shown in Fig. 9B, an elliptic hole 61 may be formed.

Moreover, as shown in Figs. 10A, 10B, and 10C, the opening 61 may be a slit in a straight line shape, a cross shape, or a Y-letter shape.

When the opening 61 is a slit, as shown in Figs. 11A or 11B, one end of a slit 61 in a straight line shape or a reversed Y-letter shape is extended to the outside of the elastic leakage protective member 60 at the upper edge on the waist hole side thereof. This portion is closed with a closing member 63 such as a tape or a Velcro zipper.

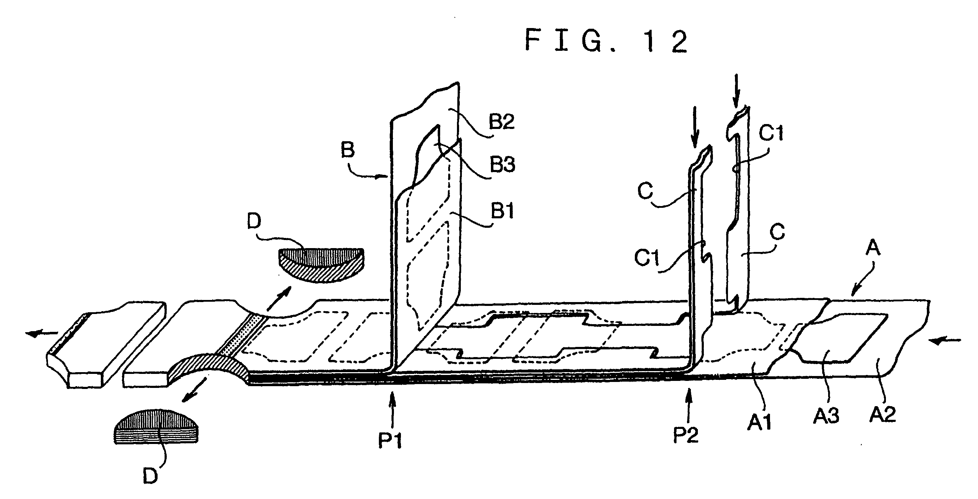

In addition, the present invention provides a method for manufacturing an absorbent article with the structure as shown in Fig. 7. Fig. 12 shows principal steps of a manufacturing process for an absorbent article according to the manufacturing method of the present invention.

In Fig. 12, letter A represents a belt-shaped first assembly which becomes front portions. Letter B represents a belt-shaped second assembly which becomes rear portions. The first assembly is composed by layering a belt-shaped top sheet A1, absorbent articles A3, and a belt-shaped back sheet A2. The absorbent articles A3 are disposed between the top sheet A1 and the back sheet A2 at predetermined intervals. The first assembly A is conveyed at a predetermined speed in an arrow direction along the longitudinal direction. Likewise, the second assembly B is composed by layering a belt-shaped top sheet B1, absorbent articles B3, and a belt-shaped back sheet B2. The absorbent articles B3 are disposed between the top sheet b1 and the back sheet B2 at predetermined intervals. The second assembly B is conveyed in the arrow direction along the longitudinal direction in synchronization with the first assembly A.

The first assembly A and the second assembly B are layered by a proper means such as a guide roller in such a manner that each absorbent article A3 faces each absorbent article B3 at a proper position P1 of the conveying path.

On the other hand, at a position P2 upstream of the position P1, a pair of sheet-shaped members C which become elastic leakage protective members are continuously supplied on the top sheet A1 of the first assembly A. Each of the sheet-shaped members C is formed by folding a belt-shaped member at the center of the width thereof. In this embodiment, openings C1 are formed from the folded edge to the opposite edge. The sheet-shaped members C are supplied to both edges of the first assembly in such a manner that the folded edges thereof face each other. A hot-melt type bonding agent supplied from a nozzle (not shown) is applied to predetermined positions of these members.

When the first assembly A and the sheet-shaped member C placed thereon reach the position P1, the second assembly B is layered on the sheet-shaped member C. The resultant layered members are supplied to a heat-bonding unit (not shown) which bonds the layered members with the hot-melt type bonding agent. At the last cutting step, unnecessary portions D are cut out and each absorbent article is separated from the layered members.

The above-described manufacturing method according to the present invention is generally referred to as longitudinal manufacturing. Since each material is conveyed in parallel with the side edge of each product, it can be manufactured at high speed. Thus, the manufacturing speed can be improved and the manufacturing cost can be reduced.

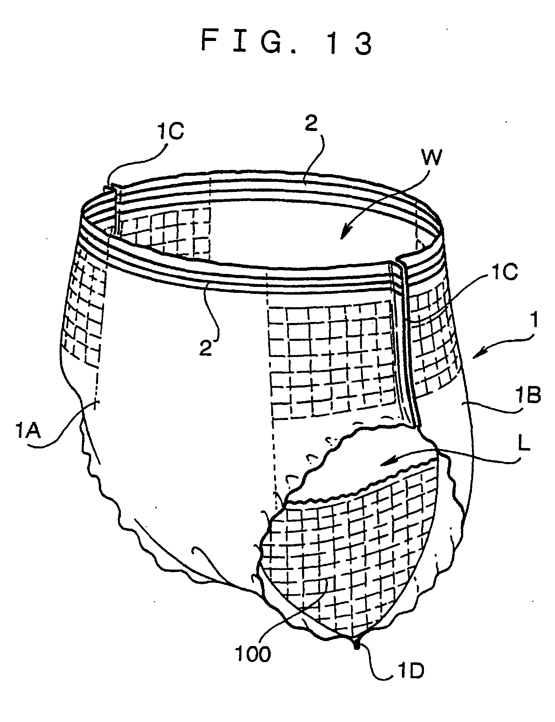

Fig. 13 is a perspective view showing an absorbent article in the form of a tape-less type diaper according to a fourth aspect of the present invention in the state that a wearer wears the absorbent article. Referring to Fig. 1, in the state that a wearer wears the absorbent article, one waist hole W and two leg holes L are formed in a main body 1 of the absorbent article. A pair of elastic leakage protective members 100 (which will be described later) are disposed in such a manner that they cover around half of the lower portion of the respective leg holes. In addition, a waist gather 2 is disposed along the waist hole W so as to provide this portion with elasticity.

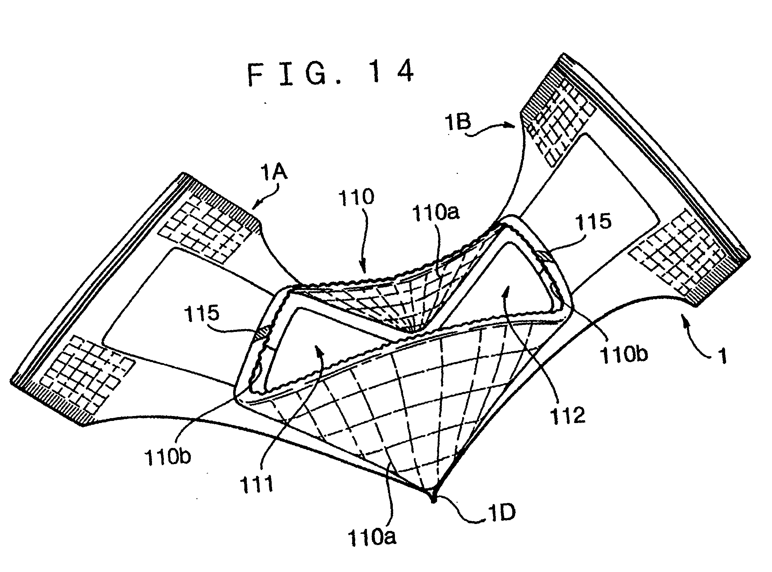

Fig. 14 is an exploded view showing an absorbent article. In Fig. 2, the main body 1 is of conventional pants-type having a liquid permeable top sheet, a liquid impermeable back sheet, and a absorbent member disposed therebetween. The main body 1 has a front portion 1A and a rear portion 1B. The front portion 1A covers the abdomen portion of the wearer. The rear portion 1B covers the hips portion. The main body 1 is formed in a rectangular shape of which the longitudinal center portion is narrowed. Each edge portion of the front portion 1A and each edge portion of the rear portion 1B are connected with respective connection portions 1C and connection portions 1D at the crotch portions of the front portion 1A and the rear portion 1B. The present invention can be applied for a tape-type absorbent article. In this case, tapes which detachably connect the front portion are disposed at both edge portions of the rear portion of the main body 1. In addition, the front portion 1A and the rear portion 1B may be integrally structured without the connection portions 1D of the crotch portions. Since the structure of the main body 1 is basically the same as the structure shown in Fig. 1, detail description thereof is omitted.

An elastic leakage protective member 110 is disposed at the center portion (namely, the crotch portion) of the main body 1. The elastic leakage protective member 110 is composed of an elastic composite sheet. The elastic composite sheet is composed of non-woven fabric and net-shaped elastic. The elastic leakage protective member 10 is formed in a prism shape. The bottom surface and two inclined surfaces of the elastic leakage protective member 110 have openings 111 and 112, respectively. The elastic leakage protective member 110 has first members 110a disposed on two opposite sides of the prism-shaped structure and second members 110b which connect the first members 110a. Lower edges of the first members 110a and the second members 110b are inwardly folded as a belt-shaped connection region. The connection region of the elastic leakage protective member 110 is connected to the inner surface of the crotch portion of the main body 1 with a suitable bonding agent such as a hot-melt type bonding agent. Reference numeral 115 is a connection portion which connects a first half portion as the first portion 110a and the second portion 110b on one side and a second half portion as the first portion 110a and the second portion 110b on the opposite side.

The material of the elastic leakage protective member 110 is the same as that of the embodiment shown in Figs. 1 and 2.

The elastic leakage protective member 110 which covers part of the leg holes L does not prevent the leg of the wearer from passing due to elasticity thereof. In the state that the wearer wears the absorbent article, the elastic leakage protective member 110 fits the legs along with the leg gathers 3, thereby stably holding the absorbent article at a predetermined position.

In addition, since the upper edge portions of the first portions 110a and the second portions 110b of the elastic leakage protective member 110 fit the crotch portion of the wearer, the elastic leakage protective member 110 provides excellent fitness and leakage protective effect. Moreover, the openings 111 and 112 formed on the two inclined surfaces of the prism-shaped structure provide paths for guiding body exudate to the absorbent member of the main body 1.

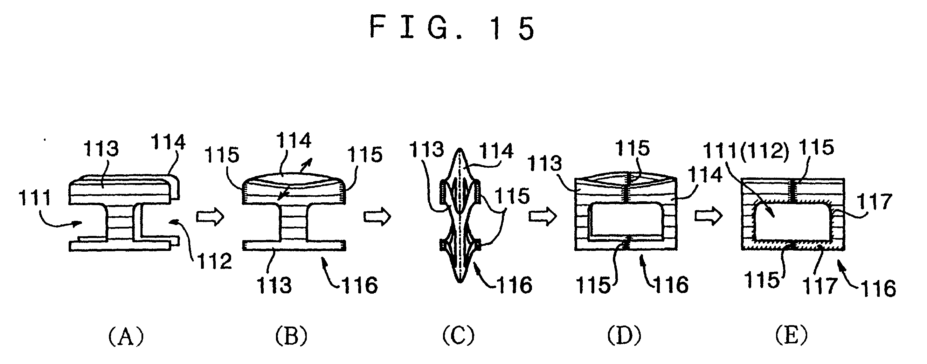

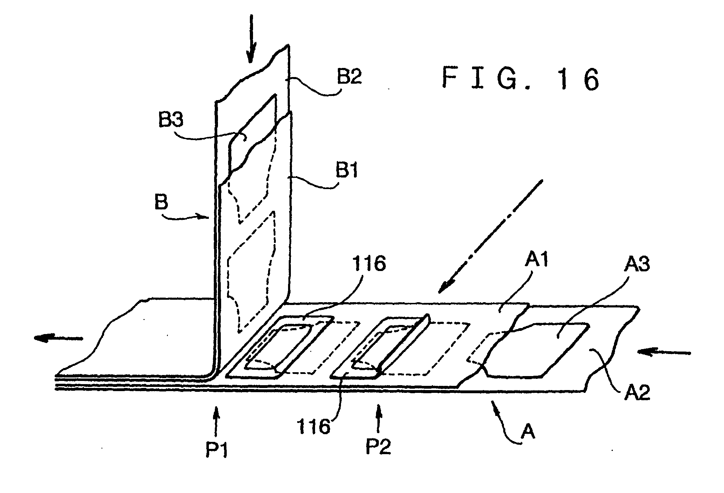

Figs. 15 and 16 show a manufacturing process for an absorbent article having the elastic leakage protective member 110 shown in Fig. 14. First of all, two I-shaped sheet members 113 and 114 which have openings 111 and 112 extending from both side edges to the center portion are prepared. The sheet members 111 and 112 are layered (at step A). Thereafter, the sheet members 111 and 112 are connected at connection portions 115 which are both the edge portions and thereby a cylindrical subassembly 116 is formed (at step B). Next, the sheet members 113 and 114 are spread out at the center position in an arrow direction in such a manner that the connection portions 115 face each other (at steps C and D). Thereafter, a hot-melt type bonding agent 117 is applied to the peripheries and the lower edges of the openings 111 and 112 of the subassembly 116 (at step E).

In the case that the connection portions 115 can be exposed to both the sides of the main body 1, at step A of Fig. 15, nearly ring-shaped members with openings at the center positions thereof are used as the sheet members 113 and 114. At step E, the hot-melt type bonding agent is applied to the predetermined positions.

The subassembly is supplied to a process shown in Fig. 16. In Fig. 16, letter A represents a first assembly which becomes front portions and is continuously supplied. Letter B represents a second assembly which becomes rear portions and is continuously supplied. The first assembly is composed by layering a belt-shaped top sheet A1, absorbent articles A3, and a belt-shaped back sheet A2. The absorbent articles A3 are disposed between the top sheet A1 and the back sheet A2 at predetermined intervals. The first assembly A is conveyed at a predetermined speed in an arrow direction along the longitudinal direction. Likewise, the second assembly B is composed by layering a belt-shaped top sheet B1, absorbent articles B3, and a belt-shaped back sheet B2. The absorbent articles B3 are disposed between the top sheet B1 and the back sheet B2 at predetermined intervals. The second assembly B is conveyed in the arrow- direction along the longitudinal direction in synchronization with the first assembly A.

The first assembly A and the second assembly B are layered by a proper means such as a guide roller in such a manner that each of the top sheets A1 contacts each of the top sheets B and that each absorbent article A3 faces each absorbent article B3 at a proper position P1 of the conveying path. On the other hand, at the position P2 upstream of the position P1, the subassemblies 116 obtained in the process shown in Fig. 15 are supplied in synchronization with the first assembly A. The subassemblies 116 are successively placed on the top sheet A1 of the first assembly A in synchronization with the absorbent members A3. Thus, when each of the subassemblies 116 reaches the position P1, the second assembly B is layered on the subassembly 116. Consequently, the subassembly 116 is sandwiched between the two assemblies A and B. A hot-melt type bonding agent has been applied to predetermined positions of the assemblies A and B.

The layered three members are supplied to a bonding step for melting the hot-melt type bonding agent and bonding the members, a cutting step for cutting out the unnecessary portions, and a separating step for separating these members into absorbent articles (these steps are not shown). Thus, final products are obtained.

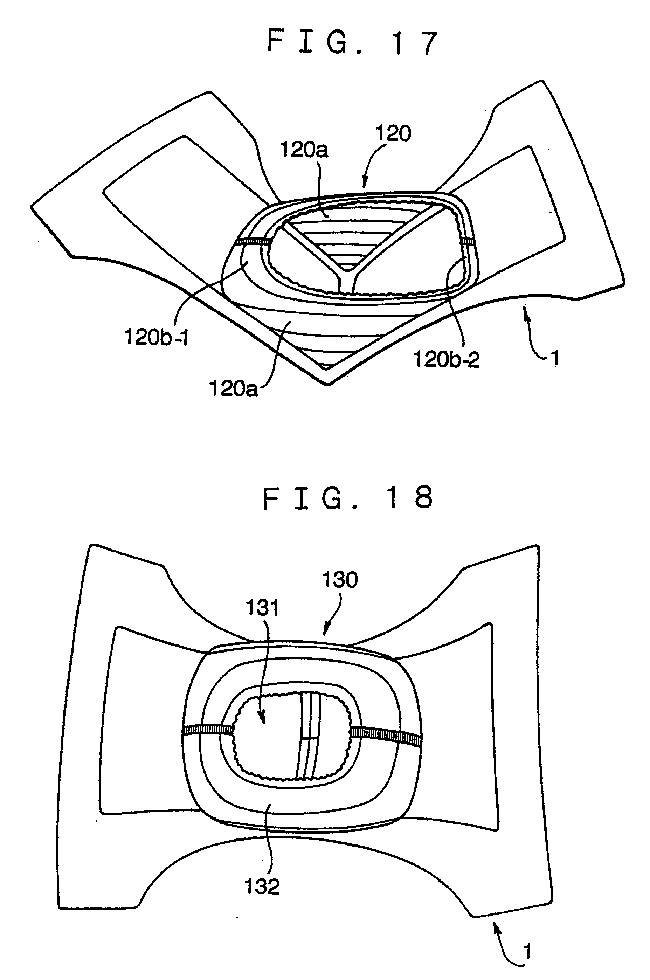

Fig. 17 shows an absorbent article according to a fifth aspect of the present invention. In Fig. 17, an elastic leakage protective member 120 disposed in a main body 1 of the absorbent article is similar to the elastic leakage protective member shown in Fig. 14. However, a second portion 120b-1 of two second portions 120b-1 and 120b-2 corresponding to the second portions 110b is wider than the other second portion 120b-2. The wider second portion 120b-1 forms a large pocket for holding body exudate along with the inner surface of the main body 1 disposed therebelow.

Fig. 18 shows an absorbent article according to a sixth aspect of the present invention. In Fig. 18, the upper edge of an elastic leakage protective member 130 extends inwardly, thereby forming a flange member 132 having an opening 131 at the center portion thereof. The opening 131 holds body exudate in the state that the wearer wears the absorbent article. The entire elastic leakage protective member 130 functions as a pocket for holding the body exudate.

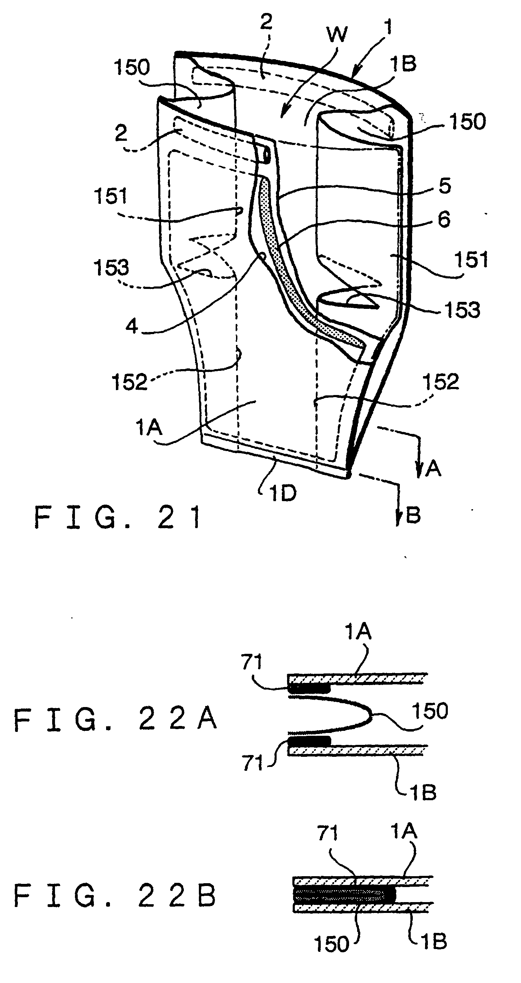

Fig. 19 shows an absorbent article according to a seventh aspect of the present invention. In Fig. 19, an elastic leakage protective member 140 disposed to a main body 1 is composed of two first portions 140a and one second portion 140b. The first portions 140a are disposed on both edges of the crotch region of the main body 1. The second portion 140b connects the first portions 140a. In other words, the second portion 140b is disposed at one of edge portions of the crotch region.

The feature of the structure shown in Fig. 19 is in that the two first portions 140a and the one second portion 140b are composed of a single elastic sheet material. The elastic leakage protective member 140 is structured by forming a nearly rectangular sheet member 141 with a proper elasticity in a U-letter shape which has part of cut-out portion 142, applying bonding agents 143 and 144 in U-letter shape on the front surface and along an outer periphery on the rear surface, placing the sheet member 141 at the center portion of the main body 1 which is two-folded, and bonding the sheet member 141 to the inner surface of the main body 1 with the bonding agents 143 and 144. Fig. 20A shows the front surface of the elastic leakage protective member 140. Fig. 20B shows the rear surface of the elastic leakage protective member.

In each of the above-described aspects (embodiments) of the present invention, the main body is composed of a top sheet, a back sheet, and an absorbent member which are different sheet materials. However, the present invention can be advantageously applied for a structure of a main body of which a front portion composed of a top sheet, a back sheet, and an absorbent member and a rear portion composed of a top sheet, a back sheet, and an absorbent member can be separately prepared and each sheet is connected at the crotch portion. In particular, when the manufacturing process shown in Figs. 15 and 16 is used, the step for connecting a front portion and a rear portion and the step for connecting them with an elastic leakage protective member can be performed at the same time and thereby the manufacturing efficiency can be improved.

Fig. 21 shows a tape-less diaper type absorbent article according to an eighth aspect of the present invention. A main body 1 of the absorbent article shown in Fig. 21 is composed of a front portion 1A which covers the front waist portion of the wearer, a rear portion 1B which covers the rear waist portion of the wearer, and a pair of elastic leakage protective members 150 which function as side panels which connect both sides of the front portion 1A and the rear portion 1B. The lower edges of the front portion 1A, the rear portion 1B, and both the leakage protective members 150 are mutually connected by a connection portion 1D. Thus, the absorbent article shown in Fig. 21 is formed in a cylindrical shape of which one end thereof is closed. The upper open end is a waist hole W.

The front portion 1A is composed of a liquid permeable top sheet 4, a liquid impermeable back sheet 5, and an absorbent member 6 disposed therebetween. The structure of the rear portion 1B is similar to that of the front portion 1A. When necessary, waist gathers 2 are disposed at the front portion 1A and the rear portion 1B along the waist hole W so as to provide these portions with elasticity.

Each of the leakage protective members 150 is composed of a sheet material which has proper elasticity at least in the lateral direction. The leakage protective member 150 is formed in a nearly rectangular shape. Before the absorbent article is used, each of the leakage protective members 150 is two-folded in the width direction (namely, lateral direction). Each of the leakage protective members 150 has an openings 153 nearly at the center position in the longitudinal direction. The opening 153 sections the leakage protective member 150 as an upper zone 151 disposed above the opening 153 and a lower zone 152 disposed below the openings 153.

As shown in Fig. 22B, the lower edge portion of each of the leakage protective members 150 is two-folded and integrally connected to the lower edge portions of the front portion 1A and the rear portion 1B at the connection portion 1D with a proper bonding agent 71 such as a hot-melt bonding agent. As shown in Fig. 22A, at other than the connection portion 1D, one side edge portion of each of the leakage protective members 150 is connected to the side edge portion of the front portion 1A with the bonding agent 71 and the other side edge portion thereof is connected to the side edge portion of the rear portion 1B with the bonding agent 71. Thus, the two-folded elastic leakage protective members 150 do not widen at the connection portion 1D, but freely spreads out at other than the connection portion 1D.

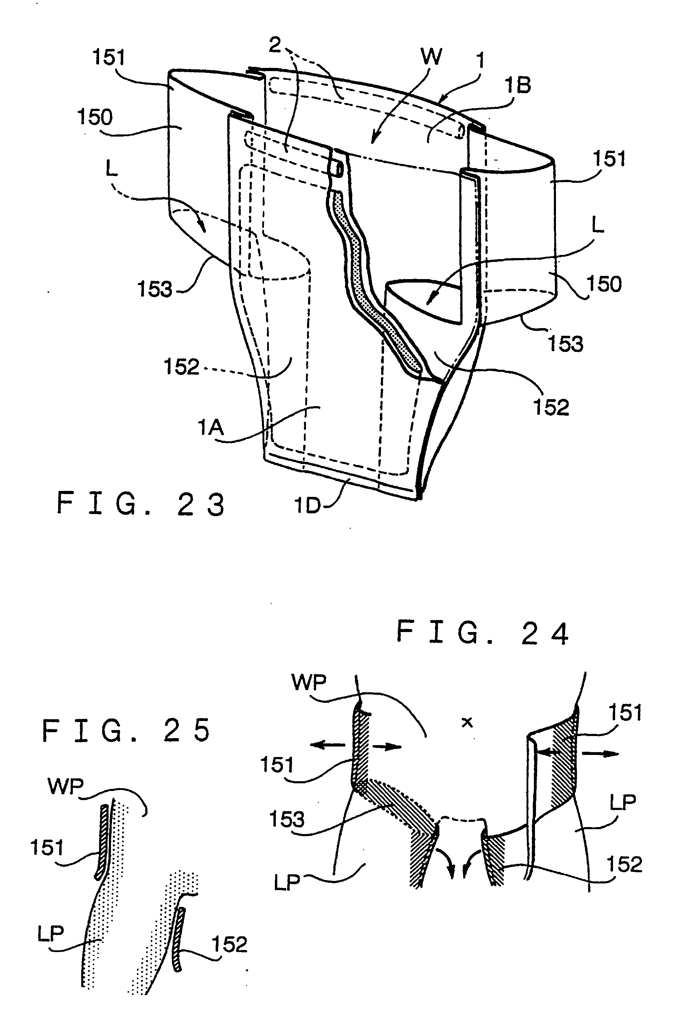

Before using the absorbent article shown in Fig. 21, only the upper zones 151 are spread out from both the side edges of the front portion 1A and the rear portion 1B as shown in Fig. 23. In this state, the upper zones 151 form a cylindrical shape along with the upper portions of the front portion 1A and the rear portion 1B. The openings 153 of the elastic leakage protective members 150 form leg holes L which are paths to the inside of the cylinder.

Figs. 24 and 25 show the contact state of the body of a wearer of the absorbent article shown in Fig. 23 and the elastic leakage protective members 150. In the state that the wearer correctly wears the absorbent article, the upper zones 151 of the elastic leakage protective members 150 are positioned on both sides of the waist portion WP of the wearer. The lower zones 152 are positioned inside the femoral portions LP. With the elasticity of the elastic leakage protective members 150, they fit these portions and the edge portions of the opening portions 153 encircle the femoral base portions.

As with conventional underpants, a wearer can wear the absorbent article of the present invention while he or she is standing. In the state that a wearer wears the absorbent article as shown in Figs. 24 and 25, the upper zones 151 fit the waist portion of the wearer. Corresponding to the motion of the wearer, the upper zones 151 securely holds the absorbent article at a predetermined position. In addition, the absorbent article does not prevent the motions of the wearer. The lower zones 152 fit the inside of the femoral base portions of the wearer, provide an excellent leakage protective effect especially required for these portions, and prevent body exudate from leaking up to their absorbing limits.

As an important feature of the absorbent article, the elastic leakage protective members 150 are sectioned as the upper zones 151 and the lower zones 152 with the openings 153. Since the upper zones 151 are vertically less restricted, after the wearer spreads out the absorbent article and wears it, the upper zones 151 expands and shrink corresponding to the motions of the waist portion of the wearer. On the other hand, the lower zones 152 are connected to the front portion and the rear portion at the crotch portion, they restrict the outward motions of the wearer, thereby maintaining a stable standing cuff state. Thus, since both the upper zones 151 and the lower zones 152 expand and shrink in opposite directions, their force does not mutually interfere. Consequently, the absorbent article provides both stable dislocation protective effect and leakage protective effect. In addition, due to absence of mutual interference, since the degree of freedom of designing the structure of an absorbent article is large, products with features corresponding to various applications can be developed.

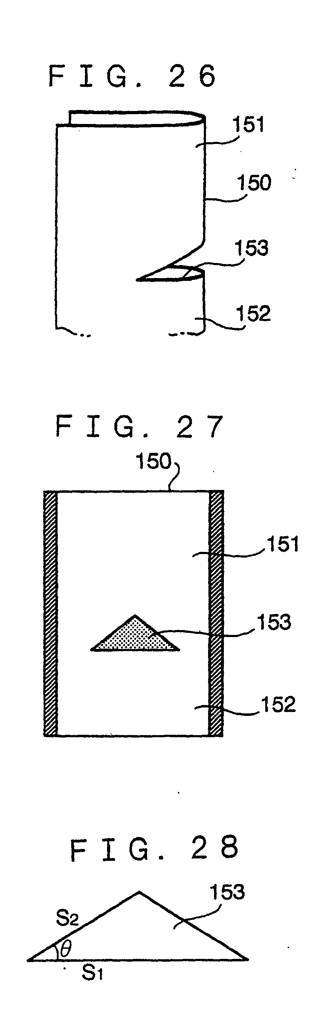

In the embodiment shown in Figs. 21 to 25, when the absorbent article is two-folded, the openings 153 in the elastic leakage protective members 150 are formed in a wedge shape as shown in Fig. 26. In the state that the absorbent article is spread out, the openings 153 in the elastic leakage protective members 150 are formed in a nearly isosceles triangle shape where the height is relatively small against the base as shown in Fig. 27. Assuming that the length of the base S1 of the isosceles triangle is constant, the area of the opening 153 is proportional to angle θ of the base S1 and the hypotenuse S2. Thus, by varying the angle θ, the size of the opening 153 can be changed.

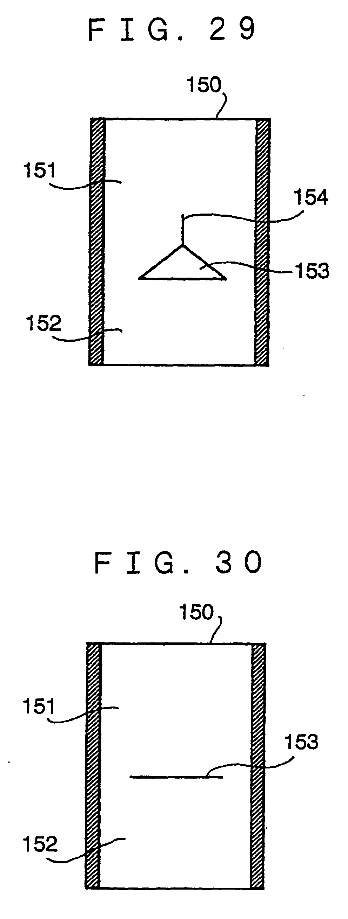

In addition, as shown in Fig. 29, an auxiliary cutting line 154 with a proper length can be formed upwardly from the vertex of the isosceles triangle in the upper zone 151. The auxiliary cutting line 154 may be a fine perforation which does not pass liquid. With the auxiliary cutting line 154, the elastic leakage protective member 150 can be easily torn from the opening 153 to the upper edge of the upper zone 151. However, when the strength necessary for tearing the elastic sheet material of the leakage protective member 150 is not too large, it can be torn without the auxiliary cutting line 154.

As shown in Fig. 30, the opening 153 may be a slit which does not substantially have a width.

As another advantage of the absorbent article shown in Figs. 21 to 30, since the leakage protective members 150 have been folded inside the front portion 1A and the rear portion 1B, a folding step is not required unlike with the conventional manufacturing method. Thus, the packaging step of products can be simplified and easily performed. In addition, the volume of a packaged product becomes very small. The products can be easily stored and transported.

As the material of the elastic leakage protective members 150 for use with such an absorbent article, the material used in the embodiment shown in Figs. 1 and 2 can be used.

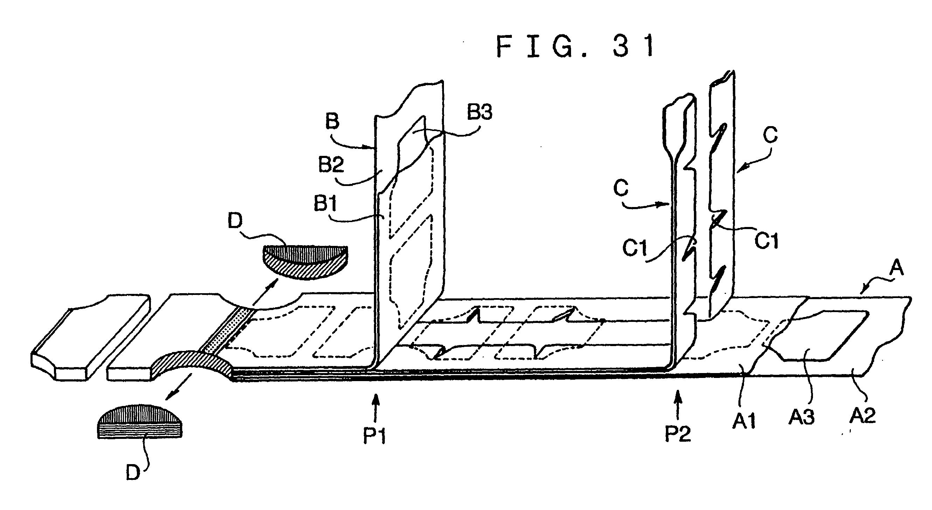

In addition, the present invention also provides a manufacturing method of the above-described absorbent article. Fig. 31 shows principal portions of the manufacturing process of the absorbent article according to the present invention.

In Fig. 31, letter A represents a belt-shaped first assembly which becomes front portions. Letter B represents a belt-shaped second assembly which becomes rear portions. The first assembly is composed by layering a belt-shaped top sheet A1, absorbent articles A3, and a belt-shaped back sheet A2. The absorbent articles A3 are disposed between the top sheet A1 and the back sheet A2 at predetermined intervals. The first assembly A is conveyed at a predetermined speed in an arrow direction along the longitudinal direction. Likewise, the second assembly B is composed by layering a belt-shaped top sheet B1, absorbent articles B3, and a belt-shaped back sheet B2. The absorbent articles B3 are disposed between the top sheet b1 and the back sheet B2 at predetermined intervals. The second assembly B is conveyed in the arrow direction along the longitudinal direction in synchronization with the first assembly A.

The first assembly A and the second assembly B are layered by a proper means such as a guide roller in such a manner that each absorbent article A3 faces each absorbent article B3 at a proper position P1 of the conveying path.

On the other hand, at a position P2 upstream of the position P1, a pair of sheet-shaped members C which become elastic leakage protective members are continuously supplied on the top sheet A1 of the first assembly A. Each of the sheet-shaped members C is formed by folding a belt-shaped member at the center of the width thereof. In this embodiment, V-shaped openings (openings C1 in the spread state) are formed from the folded edge to the opposite edge. The sheet-shaped members C are supplied to both edges of the first assembly in such a manner that the folded edges thereof face each other. A hot-melt type bonding agent supplied from a nozzle (not shown) is applied to predetermined positions of these members.

When the first assembly A and the sheet-shaped member C placed thereon reach the position P1, the second assembly B is layered on the sheet-shaped member C. The resultant layered members are supplied to a heat-bonding unit (not shown) which bonds the layered members with the hot-melt type bonding agent. At the last cutting step, unnecessary portions D are cut out and each absorbent article is separated from the layered members.

The above-described method is generally referred to as longitudinal manufacturing method. In this method, each material is conveyed in parallel with the side edges of products. However, the present invention can be also applied for lateral manufacturing method.

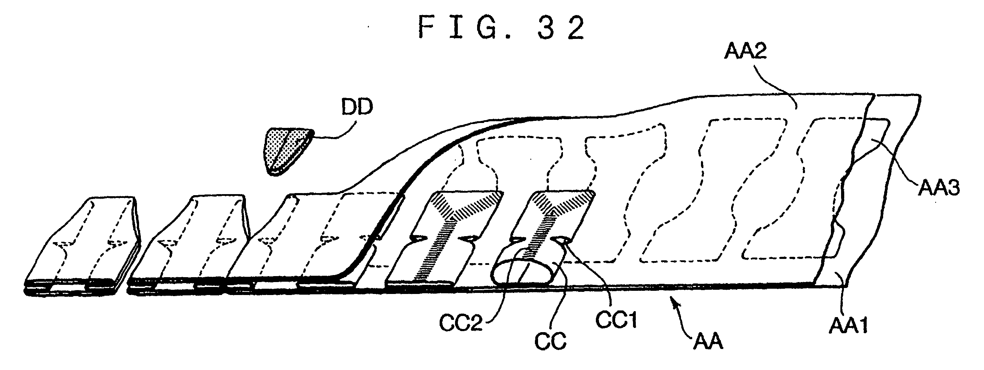

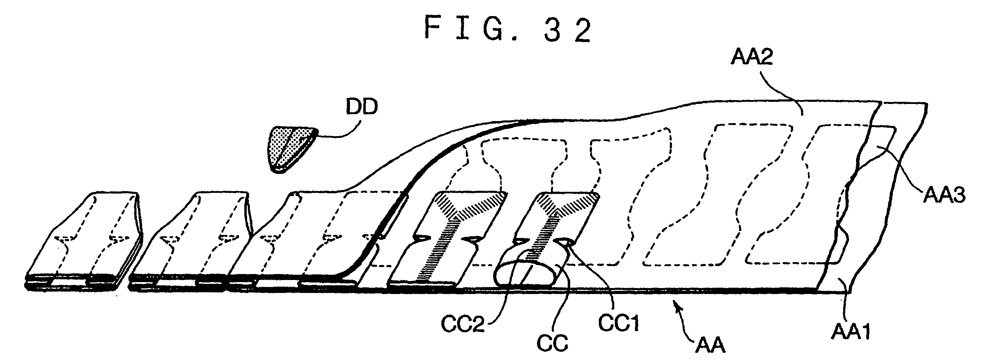

In the lateral manufacturing method, as shown in Fig. 32, a belt-shaped top sheet AA1 and a belt-shaped bottom sheet AA2 are layered. Absorbent members AA3 are placed in the non-folded state between the top sheet AA1 and the bottom sheet AA2 at predetermined intervals. Thus, a first assembly AA is formed. While the first assembly AA is being conveyed in the longitudinal direction as denoted by an arrow shown in Fig. 32 at a predetermined speed, a second assembly CC for leakage protective members composed of an elastic sheet material is supplied in such a manner that each leakage protective member is placed between absorbent members AA3. The assembly CC has slits CC1 at predetermined positions. A bonding agent CC2 such as a hot-melt type bonding agent is applied to predetermined positions of the assembly CC connected to the main body.

After the second assembly CC is placed, the first assembly is two-folded at the center portion in the width direction. Thus, the second assembly CC is sandwiched with the first assembly AA. After the first assembly AA and the second assembly CC are bonded at predetermined positions, the unnecessary portion DD is removed and the rest is cut as products.

Regardless of any of the above-described manufacturing methods, since the sheet material which composes the elastic leakage protective member which has been two-folded in the width direction are placed between two assemblies which become the front portion and the rear portion, the folding step for the products can be omitted. In addition, the volume of the products becomes small. Consequently, the products can be easily packaged and handled.

### Industrial Utilization

As described above, according to the present invention, a pair of elastic leakage protective members are disposed to a main body in such a manner that the elastic leakage protective members cover at least part of respective leg holes. The leakage protective members allow the body of a wearer to fit the absorbent article in the leg holes, thereby improving the fitness and leakage protective effect.

In addition, the absorbent articles can be effectively mass-produced by the method according to the present invention. Thus, the quality of the products can be stably maintained and the cost thereof can be reduced.

When a pair of elastic leakage protective members, each of which is composed of a first portion which closes part of a leg hole and a second portion which connects one or both edges of the first portion, are disposed on a main body, the elastic leakage protective members allow the legs of a wearer to fit the absorbent article in the leg holes and the body of the wearer to fit it in the crotch portion, thereby improving the fitness, wearing feeling, and leakage protective effect.

In addition, the absorbent articles can be effectively mass-produced by the method according to the present invention. Thus, the quality of the products can be stably maintained and the cost thereof can be reduced.

When both side edges of a front portion and a rear portion of an absorbent article are connected with a pair of elastic leakage protective members each of which is internally two-folded and the leakage protective members have openings as leg holes, each of the elastic leakage protective members is sectioned as an upper zone placed above the opening and an lower zone placed below the opening. In the state that a wearer wears this absorbent article, the upper zone is spread out and touches the waist portion of the wearer. However, the lower zone is stably kept inside and touches the inside of the femoral base portions of the wearer.

Thus, the upper zone has a fitting function for the waist portion and the outside of the femoral base portions. On the other hand, the lower zone has a fitting function for the inside of the femoral base portions. Consequently, the upper zone allows the absorbent article necessarily and satisfactorily fit to the waist portion of the wearer. The lower zone allows the absorbent article to fit the femoral base portions. Thus, the femoral base portions are inwardly surrounded by the lower edges of the upper zones and outwardly surrounded by the upper edges of the lower zones, thereby proving excellent fitness and leakage protective effect.

In addition, the absorbent article can be effectively mass-produced by the method according to the present invention. Thus, the quality of the products can be stably maintained and the cost thereof can be reduced.

## Claims

1. An absorbent article comprising a main body (1) comprising a front portion (1A), a rear portion (1B), a waist hole (W) and a pair of leg holes (L) disposed between the front portion and the rear portion, a respective elastic leakage protective member (10, 20, 30, 40, 50, 60, 100, 110, 120, 130, 140, 150) mounted at a site corresponding to each leg hole of the main body to interconnect edge portions of the front and rear portions so that at least part of the respective leg hole is closed, **characterised in that** each of the leakage protective members has an area accounting for at least two thirds of the area of the respective leg hole.

2. An absorbent article comprising a main body (1) comprising a front portion (1A), a rear portion (1B), a waist hole (W) and a pair of leg holes (L) disposed between the front portion and the rear portion, a respective elastic leakage protective member (10, 20, 30, 40, 50, 100, 110, 120, 130, 140, 150) mounted at a site corresponding to each leg hole of the main body to interconnect edge portions of the front and rear portions so that at least part of the respective leg hole is closed, **characterised in that** each of the leakage protective members has a width which is widest at a part lying at the centre of the thigh portion of the main body, and which becomes narrower at the waist hole side than that.

3. An absorbent article according to claim 1 or claim 2, wherein the front portion (1A) and the rear portion (1B) are formed integrally.

4. An absorbent article according to claim 1 or claim 2, wherein the main body (1) is formed by connecting the front portion (1A) and the rear portion (1B) at crotch portions thereof.

5. An absorbent article according to any one of claims 1 to 4, wherein the leakage protective member (30) has a slit (31) which terminates at an outside edge thereof

6. An absorbent article according to claim 5, wherein the top edge portion of the slit (31) is split into a plurality of portions.

7. An absorbent article according to claim 2, wherein the leakage protective members (10, 20, 30, 40, 50, 100, 110, 120, 130, 140, 150) are mounted to the main body to couple between edge portions of the front portion and the rear portion, the front portion, the rear portion and the upper edge portions of the leakage protective members being annularly continuous to form the waist hole (W), respective openings which serve as the leg holes (L) being formed at each of the leakage protective members.

8. An absorbent article according to claim 7, wherein each opening is of elongated elliptical form whose longer axis is disposed in the vertical direction.

9. An absorbent article according to claim 7, wherein each opening is of elongated elliptical form whose longer axis is disposed in the horizontal direction.

10. An absorbent article according to claim 7, wherein each opening is in the form an equilateral triangle with its base oriented horizontally.

11. An absorbent article according to claim 10, wherein each opening is in the form an equilateral triangle with is base oriented horizontally and has a slit extending upwardly from the apex of the triangle.

12. An absorbent article according to claim 11, wherein the slit is of horizontally-extending rectilinear form.

13. An absorbent article according to claim 11, wherein the slit is of a cross form.

14. An absorbent article according to claim 11, wherein the slit is of a Y-shaped form.

15. An absorbent article according to any one of claims 7 to 14, wherein each leakage protective member has no joining member other that between both side edge portions.

16. An absorbent article according to any one of claims 12 to 14, wherein the slit of each leakage protective member reaches the upper edge portion of that leakage protective member at the edge portion which is closed by means of coupling members mounted to that leakage protective member.

17. The absorbent article according to any one of claims 1 to 16, wherein the main body has a structure in which the separately-formed front and rear portions are coupled at each crotch portion.

18. An absorbent article according to claim 2, wherein each elastic leakage protective member (140) comprises first and second portions (140a, 140b), the first portions being attached to the main body so as to block at least part of each leg hole, and each second portion being coupled to at least part of one of the first portions and to at least part of the other of the first portions.

19. An absorbent article according to claim 18, wherein the second portion is provided only at part of the first portion.

20. An absorbent article according to claim 18, wherein the second portion is provided only at part of the both edges of the first portion.

21. An absorbent article according to any one of claims 18 to 20, wherein the second portion extends upwardly to an absorbing area formed above an absorbent member disposed at the centre of the main body to form a pocket between the second portion and the main body.

22. An absorbent article according to claim 21, wherein the elastic leakage protective member is made of a substantially U-shaped elastic sheet material having a cut-out portion, and is joined to the inner surface of the main body at one surface of the sheet material at the edge portion of the cut-out portion, and to the other surface at both outer edge portions thereof.

23. A method of manufacturing an absorbent article having a main body (1) comprising a front portion (1A) and a rear portion (1B), the method comprising the steps of:
conveying a first continuous elongated strip-like assembly prepared for forming the front portion, in its longitudinal direction;
supplying continuously to both edges of the first assembly (A) a pair of sheet materials each forming a continuous strip-like member (C) folded into two layers at its centre as viewed in its width direction and the width direction of the first continuous elongated strip assembly, and having openings (C1) formed at a predetermined interval;
superimposing a second continuous strip-like assembly (B), which serves as the rear portion, onto the first assembly with the sheet-shaped material superimposed thereon while conveying in its longitudinal direction;
joining the first assembly, the second assembly and the sheet material at a predetermined position for integration; and
cutting off the integrated first assembly, the second assembly and the sheet-shaped material at a predetermined interval to obtain a discrete absorbent article, in which each of the pair of sheet materials interconnects edge portions of the first assembly and the second assembly such that the openings in the sheet materials form leg holes.

24. The method according to claim 23, wherein the step of joining is carried out by means of a hot melt type adhesive agent applied at a predetermined position.

25. An absorbent article having a front portion (1A) and a rear portion (1B) interconnected together at side edge portions thereof, **characterised in that** the article comprises:
leakage protective members (150) of a resilient sheet material interconnecting each facing side edge portions of the front portion and the rear portion over the entire length extending from the side edge portion of the waist up to the side edge portion of the crotch portion; and
each leakage protective member being divided into an upper zone (151) and a lower zone (152) by means of an opening (153) formed at a predetermined position of the area extending from its upper edge up to its lower edge;
whereby the opening of each leakage protective member functions as a leg hole (L) while one wears this article, with the wearer's leg inserted into the leg hole, the upper zone is developed outwardly so as to come in contact with the wearer's waist portion, and the lower zone is developed inwardly so as to come in contact with the inner side of the wearer's leg.

26. An absorbent article in which a front portion (1A) and a rear portion (1B) are separately formed, and their side edge portions and the crotch portion are interconnected, **characterised in that** the article comprises:
leakage protective members (150) which join the front to the rear portion of the absorbent article and each of which is divided into an upper zone (151) and a lower zone (152) , being folded inwardly relative to the central portion of the main body in its width direction whereby the front portion and the rear portion are folded so as to face each other;
whereby when being worn the upper zone is developed outwardly so as to come in contact with the wearer's waist portion, and the lower zone is developed inwardly so as to come in contact with the inner side of the wearer's leg.

27. An absorbent article according to claim 25 or claim 26, wherein the leakage protection member comprises a seamless sheet material of a single structure.

28. : A absorbent article according to any one of claims 25 to 27, wherein the opening is a slit extending in the width direction of the leakage protective member.

29. An absorbent article according to any one of claims 25 to 28, wherein the opening takes the form of a substantially equilateral triangle with a straight line extending in the width direction taken as its bottom side.

30. An absorbent article according to any one of claims 25 to 29, wherein the lower zone of the leakage protective member is mutually joined to the front portion and the rear portion at the lower edge portion, where it is folded inwardly at the central portion as viewed in its width direction thereby to prevent the outward deformation of the lower zone.

31. A method of manufacturing an absorbent article having front and rear portions (1A and 1B) interconnected at side edge portions and the crotch portions thereof, leakage protective members (150) made of an elastic sheet material interconnecting the facing side edge portions of the front and rear portions over the entire length extending from the side edge portion of the waist up to the side edge portion of the crotch portion, each leakage protective member being divided into an upper zone (151) and a lower zone (152) by means of a leg opening (153) formed at a predetermined position of an area extending from its upper edge up to its lower edge, the method comprising the steps of:
superimposing continuous strip-like top and back sheets (A1 and A2) to prepare a first assembly formed with an absorbent member (A3) therebetween at a proper interval, the first assembly serving as the front portion;
superimposing continuous strip-like top and back sheets (B1 and B2) to prepare a second assembly formed with an absorbent member (B3) sandwiched therebetween at a proper interval, the second assembly serving as the rear portion;
continuously supplying the first and second assemblies at a predetermined point (P1) of their transfer path at a speed synchronised so as to be superimposed at such a timing at which each absorbent member faces each other;
folding a continuous sheet material (C), which serves as the leakage protective member, into two at the central portion as viewed in its width direction and forming the opening (C1) at a predetermined interval to form a member adapted for the leakage protective member; continuously supplying a sub-assembly between the first and second assemblies, folded into two at a point (P2) lying ahead of the point (P1);
bonding the first and second assemblies and the member adapted for the leakage protective member at a predetermined binding portion; and
cutting the first and second assemblies along a predetermined cutting line to separate into individual absorbent articles.

## Patentansprüche

1. Saugfähiger Artikel mit einem Hauptkörper (1) aus einem Vorderteil (1A) einem Rückteil (1B), einem Hüftloch (W) und einem Paar zwischen dem Vorderteil und dem Rückteil angeordneter Beinlöcher (L), wobei ein jeweiliges elastisches Auslaufschutzelement (10, 20, 30, 40, 50, 60, 100, 110, 120, 130, 140, 150) an einer Stelle angebracht ist, die jedem Beinloch des Hauptkörpers entspricht, um Randteile des Vorder- und des Rückteils miteinander zu verbinden, damit mindestens ein Teil des jeweiligen Beinlochs geschlossen ist, **dadurch gekennzeichnet, dass** die Fläche jedes Auslaufschutzelements mindestens zwei Dritteln der Fläche des jeweiligen Beinlochs entspricht.

2. Saugfähiger Artikel mit einem Hauptkörper (1) aus einem Vorderteil (1A), einem Rückteil (1B), einem Hüftloch (W) und einem Paar zwischen dem Vorderteil und dem Rückteil angeordneter Beinlöcher (L), wobei ein jeweiliges elastisches Auslaufschutzelement (10, 20, 30, 40, 50, 100, 110, 120, 130, 140, 150) an einer Stelle angebracht ist, die jedem Beinloch des Hauptkörpers entspricht, um Randteile des Vorder- und des Rückteils miteinander zu verbinden, damit mindestens ein Teil des jeweiligen Beinlochs geschlossen ist, **dadurch gekennzeichnet, dass** jedes der Auslaufschutzelemente eine Breite hat, die an einem in der Mitte des Oberschenkelteils des Hauptkörpers liegenden Teil am größten ist und an der Hüftlochseite vergleichsweise schmaler wird.

3. Saugfähiger Artikel nach Anspruch 1 oder 2, bei dem der Vorderteil (1A) und der Rückteil (1B) einstückig gebildet sind.

4. Saugfähiger Artikel nach Anspruch 1 oder 2, bei dem der Hauptkörper (1) gebildet wird, indem man den Vorderteil (1A) und den Rückteil (1B) an ihren Schrittteilen verbindet.

5. Saugfähiger Artikel nach einem der Ansprüche 1 bis 4, bei dem das Auslaufschutzelement (30) einen Schlitz (31) aufweist, der an einem Außenrand davon endet.

6. Saugfähiger Artikel nach Anspruch 5, bei dem der obere Randteil des Schlitzes (31) in mehrere Teile geteilt ist.

7. Saugfähiger Artikel nach Anspruch 2, bei dem die Auslaufschutzelemente (10, 20, 30, 40, 50, 100, 110, 120, 130, 140, 150) am Hauptkörper angebracht sind, um die Randteile des Vorderteils und des Rückteils zu verbinden, wobei der Vorderteil, der Rückteil und die oberen Randteile der Auslaufschutzelemente zur Bildung des Hüftlochs (W) ringförmig durchgehend sind und an jedem der Auslaufschutzelemente jeweilige als die Beinlöcher (L) dienende Öffnungen gebildet werden.

8. Saugfähiger Artikel nach Anspruch 7, bei dem jede Öffnung die Form einer länglichen Ellipse hat, deren längere Achse in der vertikalen Richtung angeordnet ist.

9. Saugfähiger Artikel nach Anspruch 7, bei dem jede Öffnung die Form einer länglichen Ellipse hat, deren längere Achse in der horizontalen Richtung angeordnet ist.

10. Saugfähiger Artikel nach Anspruch 7, bei dem jede Öffnung die Form eines gleichseitigen Dreiecks hat, dessen Basis horizontal ausgerichtet ist.

11. Saugfähiger Artikel nach Anspruch 10, bei dem jede Öffnung die Form eines gleichseitigen Dreiecks hat, dessen Basis horizontal ausgerichtet ist, und einen sich von dem Scheitel des Dreiecks nach oben erstreckenden Schlitz aufweist.

12. Saugfähiger Artikel nach Anspruch 11, bei dem der Schlitz eine sich horizontal erstreckende, geradlinige Form hat.

13. Saugfähiger Artikel nach Anspruch 11, bei dem der Schlitz kreuzförmig ist.

14. Saugfähiger Artikel nach Anspruch 11, bei dem der Schlitz Y-förmig ist.

15. Saugfähiger Artikel nach einem der Ansprüche 7 bis 14, bei dem jedes Auslaufschutzelement als Verbindungselement nur das zwischen beiden Seitenrandteilen vorliegende aufweist.

16. Saugfähiger Artikel nach einem der Ansprüche 12 bis 14, bei dem der Schlitz jedes Auslaufschutzelements an dem Randteil, der durch an diesem Auslaufschutzelement angebrachte Koppelelemente geschlossen ist, den oberen Randteil dieses Auslaufschutzelements erreicht.

17. Saugfähiger Artikel nach einem der Ansprüche 1 bis 16, bei dem der Hauptkörper eine Struktur aufweist, bei der der separat gebildete Vorder- und Rückteil an jedem Schrittteil gekoppelt sind.

18. Saugfähiger Artikel nach Anspruch 2, bei dem jedes elastische Auslaufschutzelement (140) erste und zweite Teile (140a, 140b) umfasst, wobei die ersten Teile so am Hauptkörper angebracht sind, dass sie jedes Beinloch mindestens teilweise blockieren, und jeder zweite Teil an mindestens einen Teil eines der ersten Teile und an mindestens einen Teil des anderen der ersten Teile gekoppelt ist.

19. Saugfähiger Artikel nach Anspruch 18, bei dem der zweite Teil nur an einem Teil des ersten Teils vorgesehen ist.

20. Saugfähiger Artikel nach Anspruch 18, bei dem der zweite Teil nur an einem Teil der beiden Ränder des ersten Teils vorgesehen ist.

21. Saugfähiger Artikel nach einem der Ansprüche 18 bis 20, bei dem sich der zweite Teil nach oben zu einem saugfähigen Bereich erstreckt, der über einem saugfähigen Element gebildet ist, das in der Mitte des Hauptkörpers angeordnet ist, um eine Tasche zwischen dem zweiten Teil und dem Hauptkörper zu bilden.

22. Saugfähiger Artikel nach Anspruch 21, bei dem das elastische Auslaufschutzelement aus einem im Wesentlichen U-förmigen elastischen Flächenmaterial mit einem ausgeschnittenen Teil hergestellt und mit der Innenfläche des Hauptkörpers an einer Fläche des Flächenmaterials am Randteil des ausgeschnittenen Teils und mit der anderen Fläche an beiden äußeren Randteilen davon verbunden ist.

23. Verfahren zur Herstellung eines saugfähigen Artikels mit einem Hauptkörper (1) aus einem Vorderteil (1A) und einem Rückteil (1B), bei dem man eine erste durchgehende längliche, streifenartige zur Bildung des Vorderteils vorbereitete Anordnung in ihre Längsrichtung fördert,
beiden Rändern der ersten Anordnung (A) durchgehend ein Paar Flächenmaterialien zuführt, die jeweils ein durchgehendes streifenartiges Element (C) bilden, das in seiner Breitenrichtung und in der Breitenrichtung der ersten durchgehenden länglichen Streifenanordnung gesehen in seiner Mitte in zwei Lagen gefaltet ist und in einem vorbestimmten Abstand gebildete Öffnungen (C1) aufweist,
eine zweite durchgehende streifenartige, als Rückteil dienende Anordnung (B) auf die erste Anordnung legt, wobei das flächenförmige Material daraufliegt, während man sie in ihre Längsrichtung fördert,
die erste Anordnung, die zweite Anordnung und das Flächenmaterial an einer vorbestimmten Integrationsposition miteinander verbindet und
die erste Anordnung, die zweite Anordnung und das flächenförmige Material, die integriert wurden, in einem vorbestimmten Abstand abschneidet und so einen getrennten saugfähigen Artikel erhält, bei dem jedes des Paars Flächenmaterialien Randteile der ersten Anordnung und der zweiten Anordnung so miteinander verbindet, dass die Öffnungen in den Flächenmaterialien Beinlöcher bilden.

24. Verfahren nach Anspruch 23, bei dem der Schritt des Verbindens ausgeführt wird, indem man einen Schmelzkleber an einer vorbestimmten Position aufbringt.

25. Saugfähiger Artikel, der einen Vorderteil (1A) und einen Rückteil (1B) aufweist, die an ihren Seitenrandteilen miteinander verbunden sind, **dadurch gekennzeichnet, dass** der Artikel
aus einem elastischen Flächenmaterial hergestellte Auslaufschutzelemente (150) umfasst, die jeweils sich gegenüberliegende Seitenrandteile des Vorderund Rückteils über die gesamte Länge vom Seitenrandteil der Hüfte bis zum Seitenrandteil des Schrittteils miteinander verbinden,
wobei jedes Auslaufschutzelement durch eine an einer vorbestimmten Position des Bereichs von seinem oberen Rand bis zu seinem unteren Rand gebildete Öffnung (153) in eine obere Zone (151) und eine untere Zone (152) unterteilt ist,
wobei die Öffnung jedes Auslaufschutzelements als ein Beinloch (L) fungiert, während man diesen Artikel trägt, und die obere Zone nach außen entwickelt ist, um in Kontakt mit dem Hüftteil des Trägers zu kommen, und die untere Zone nach innen entwickelt ist, um in Kontakt mit der Innenseite des Beins des Trägers zu kommen, wenn der Träger die Beine durch die Beinlöcher gesteckt hat.

26. Saugfähiger Artikel, bei dem ein Vorderteil (1A) und ein Rückteil (1B) separat gebildet sind und ihre Seitenrandteile und der Schrittteil miteinander verbunden sind, **dadurch gekennzeichnet, dass** der Artikel Folgendes umfasst:
Auslaufschutzelemente (150), die den Vorder- mit dem Rückteil des saugfähigen Artikels verbinden und jeweils in eine obere Zone (151) und eine untere Zone (152) unterteilt sind, die in ihrer Breitenrichtung gesehen bezüglich des mittleren Teils des Hauptkörpers nach innen gefaltet sind,
wobei der Vorderteil und der Rückteil so gefaltet sind, dass sie einander gegenüberliegen;
wobei die obere Zone im getragenen Zustand nach außen entwickelt ist, sodass sie mit dem Hüftteil des Trägers in Kontakt kommt, und die untere Zone nach innen entwickelt ist, sodass sie mit der Innenseite des Beins des Trägers in Kontakt kommt.

27. Saugfähiger Artikel nach Anspruch 25 oder 26, bei dem das Auslaufschutzelement ein nahtloses Flächenmaterial mit einer einzigen Struktur umfasst.

28. Saugfähiger Artikel nach einem der Ansprüche 25 bis 27, bei dem es sich bei der Öffnung um einen sich in der Breitenrichtung des Auslaufschutzelements erstreckenden Schlitz handelt.

29. Saugfähiger Artikel nach einem der Ansprüche 25 bis 28, bei dem die Öffnung die Form eines im Wesentlichen gleichseitigen Dreiecks hat, wobei eine sich in der Breitenrichtung erstreckende gerade Linie seine Bodenseite bildet.

30. Saugfähiger Artikel nach einem der Ansprüche 25 bis 29, bei dem die untere Zone des Auslaufschutzelements mit dem Vorderteil und dem Rückteil am unteren Randteil verbunden ist, wo sie in ihrer Breitenrichtung gesehen am mittleren Teil nach innen gefaltet ist, um dadurch die Auswärtsdeformierung der unteren Zone zu verhindern.

31. Verfahren zur Herstellung eines saugfähigen Artikels, der einen Vorderteil und einen Rückteil (1A und 1B), die an ihren Seitenrandteilen und Schrittteilen miteinander verbunden sind, aufweist, wobei aus einem elastischen Flächenmaterial hergestellte Auslaufschutzelemente (150) die sich gegenüberliegenden Seitenrandteile des Vorder- und Rückteils über die gesamte Länge vom Seitenrandteil der Hüfte bis zum Seitenrandteil des Schrittteils miteinander verbinden und jedes Auslaufschutzelement durch eine an einer vorbestimmten Position eines Bereichs von seinem oberen Rand bis zu seinem unteren Rand gebildete Beinöffnung (153) in eine obere Zone (151) und eine untere Zone (152) unterteilt ist, wobei man bei dem Verfahren
eine durchgehende streifenartige Deckfläche und Rückfläche (A1 und A2) übereinander legt, um eine mit einem in einem geeigneten Abstand dazwischen eingelegten saugfähigen Element (A3) gebildete erste Anordnung herzustellen, wobei die erste Anordnung als Vorderteil dient,
eine durchgehende streifenartige Deckfläche und Rückfläche (B1 und B2) übereinander legt, um eine mit einem in einem geeigneten Abstand dazwischen eingelegten saugfähigen Element (B3) gebildete zweite Anordnung herzustellen, wobei die zweite Anordnung als Rückteil dient,
die erste und die zweite Anordnung an einem vorbestimmten Punkt (P1) ihrer Transferbahn mit einer Geschwindigkeit durchgehend zuführt, die so synchronisiert ist, dass das Übereinanderlegen zu dem Zeitpunkt erfolgt, an dem jedes saugfähige Element dem jeweils anderen gegenüberliegt,
ein durchgehendes, als das Auslaufschutzelement dienendes Flächenmaterial (C) am in seiner Breitenrichtung gesehen mittleren Teil in zwei Hälften faltet und zur Bildung eines für das Auslaufschutzelement geeigneten Elements die Öffnung (C1) an einem vorbestimmten Abstand bildet,
eine Unteranordnung durchgehend zwischen die erste und die zweite Anordnung zuführt, und zwar an einem Punkt (P2), der vor dem Punkt (P1 liegt, in zwei Hälften gefaltet,
die erste und die zweite Anordnung und das für das Auslaufschutzelement geeignete Element an einem vorbestimmten Bindeteil verklebt und
die erste und die zweite Anordnung entlang einer vorbestimmten Schnittlinie schneidet und in einzelne saugfähige Artikel trennt.

## Revendications

1. Article absorbant comprenant un corps principal (1) comprenant une partie avant (1A), une partie arrière (1B), un trou (W) pour la taille et une paire de trous (L) pour les jambes qui sont disposés entre la partie avant et la partie arrière, un organe de protection de fuite élastique respectif (10, 20, 30, 40, 50, 60, 100, 110, 120, 130, 140, 150) monté en un endroit correspondant à chaque trou de jambe du corps principal de manière à relier les parties de bord des parties avant et arrière de façon qu'au moins une partie du trou de jambe respectif soit obturée, **caractérisé en ce que** chacun des organes de protection de fuite présente une surface correspondant à au moins deux tiers de la surface du trou de jambe respectif.

2. Article absorbant comprenant un corps principal (1) comprenant une partie avant (1A), une partie arrière (1B), un trou (W) pour la taille et une paire de trous (L) pour les jambes qui sont disposés entre la partie avant et la partie arrière, un organe de protection de fuite élastique respectif (10, 20, 30, 40, 50, 60, 100, 110, 120, 130, 140, 150) monté en un endroit correspondant à chaque trou de jambe du corps principal de manière à relier les parties de bord des parties avant et arrière de façon qu'au moins une partie du trou de jambe respectif soit obturée, **caractérisé en ce que** chacun des organes de protection de fuite présente une largeur qui est plus large dans une partie se situant au centre de la partie de cuisse du corps principal, et qui devient plus étroite du côté du trou pour la taille que celle-là.

3. Article absorbant selon la revendication 1 ou la revendication 2, dans lequel la partie avant (1A) et la partie arrière (1B) sont formées d'une seule pièce.

4. Article absorbant selon la revendication 1 ou la revendication 2, dans lequel le corps principal (1) est formé en reliant la partie avant (1A) et la partie arrière (1B) dans leurs parties d'entrejambe.

5. Article absorbant selon l'une quelconque des revendications 1 à 4, dans lequel l'organe de protection de fuite (30) présente une fente (31) qui se termine sur l'un de ses bords extérieurs.

6. Article absorbant selon la revendication 5, dans lequel la partie de bord de dessus de la fente (31) est divisée en une pluralité de parties.

7. Article absorbant selon la revendication 2, dans lequel les organes de protection de fuite (10, 20, 30, 40, 50, 60, 100, 110, 120, 130, 140, 150) sont montés sur le corps principal de façon à réaliser le couplage entre les parties de bord de la partie avant et de la partie arrière, la partie avant, la partie arrière et les parties de bord supérieur des organes de protection de fuite étant continus et annulaires de manière à former le trou (W) pour la taille, les ouvertures respectives qui servent de trous (L) pour les jambes étant formées sur chaque organe de protection de fuite.

8. Article absorbant selon la revendication 7, dans lequel chaque ouverture a une forme elliptique allongée dont l'axe le plus long est dirigé dans la direction verticale.

9. Article absorbant selon la revendication 7, dans lequel chaque ouverture a une forme elliptique allongée dont l'axe le plus long est dirigé dans la direction horizontale.

10. Article absorbant selon la revendication 7, dans lequel chaque ouverture a la forme d'un triangle équilatéral dont la base est orientée horizontalement.

11. Article absorbant selon la revendication 10, dans lequel chaque ouverture a la forme d'un triangle équilatéral dont la base est orientée horizontalement et comporte une fente qui s'étend vers le haut à partir du sommet du triangle.

12. Article absorbant selon la revendication 11, dans lequel la fente a une forme rectiligne s'étendant horizontalement.

13. Article absorbant selon la revendication 11, dans laquelle la fente a la forme d'une croix.

14. Article absorbant selon la revendication 11, dans laquelle la fente a la forme d'un Y.

15. Article absorbant selon l'une quelconque des revendications 7 à 14, dans lequel chaque organe de protection de fuite ne comporte pas d'organe de jonction autre que celui qui se trouve entre les deux parties de bord latéral.

16. Article absorbant selon l'une quelconque des revendications 12 à 14, dans lequel la fente de chaque organe de protection de fuite atteint la partie de bord supérieur de cet organe de protection de fuite au niveau de la partie de bord qui est obturée au moyen des organes de couplage montés sur cet organe de protection de fuite.

17. Article absorbant selon l'une quelconque des revendications 1 à 16, dans lequel le corps principal a une structure dans laquelle les parties formées séparément avant et arrière sont couplées à chaque partie d'entrejambe.

18. Article absorbant selon la revendication 2, dans lequel chaque organe de protection de fuite élastique (140) comprend une première et une seconde parties (140a, 140b), les premières parties étant fixées au corps principal de façon à bloquer au moins une partie de chaque trou de jambe, et chaque seconde partie étant couplée à au moins une partie de l'une des premières parties et à au moins une partie de l'autre des premières parties.

19. Article absorbant selon la revendication 18,dans lequel la seconde partie est prévue sur seulement une partie de la première partie.

20. Article absorbant selon la revendication 18, dans lequel la seconde partie est prévue sur seulement une partie des deux bords de la première partie.

21. Article absorbant selon l'une quelconque des revendications 18 à 20, dans lequel la seconde partie s'étend vers le haut jusqu'à une région d'absorption formée au-dessus d'un organe absorbant disposé au centre du corps principal pour former une poche entre la seconde partie et le corps principal.

22. Article absorbant selon la revendication 21, dans lequel l'organe de protection de fuite élastique est fabriqué en un matériau en feuille élastique sensiblement en forme de U comportant une partie de découpe, et il est relié à la surface intérieure du corps principal sur une surface du matériau en feuille dans une partie de bord de la partie de découpe, et sur l'autre surface à ses deux parties de bord extérieur.

23. Procédé de fabrication d'un article absorbant comportant un corps principal (1) comprenant une partie avant (1A) et une partie arrière (1B), le procédé comprenant les étapes consistant à :
convoyer un première ensemble de type bande allongée continue préparé pour former la partie avant, dans sa direction longitudinale ;
fournir de façon continue aux deux bords du premier ensemble (A) une paire de matériaux en feuille formant chacun un organe continu de type bande (C) plié en deux couches en son centre lorsqu'on regarde dans la direction de sa largeur et de la direction en largeur du premier ensemble de type bande allongée continue, et comportant des ouvertures (C1) formées avec un intervalle prédéterminé ;
superposer un second ensemble (B) de type bande allongée continue, qui sert de partie arrière, sur le premier ensemble avec le matériau en forme de feuille superposé par-dessus tout en le convoyant dans sa direction longitudinale ;
réunir le premier ensemble, le second ensemble et la feuille de matériau en une position prédéterminée pour leur intégration ; et
découper le premier ensemble intégré, le second ensemble et le matériau en forme de feuille à des intervalles prédéterminés de façon à obtenir un article absorbant discret, dans lequel chacune des paires de matériaux en feuille relie les parties de bord du premier ensemble et du second ensemble de telle façon que les ouvertures formées dans les matériaux en feuille forment les trous de jambe.

24. Procédé selon la revendication 23, dans lequel l'étape de jonction est effectuée au moyen d'un agent adhésif de type fondu à chaud appliqué en une position prédéterminée.

25. Article absorbant comportant une partie avant (1A) et une partie arrière (1B) reliées entre elles sur leurs parties de bord, **caractérisé en ce que** l'article comprend :
des organes de protection de fuite (150) en un matériau en feuille résilient qui relie chacune des parties de bord latéral se faisant face de la partie avant et de la partie arrière sur toute la longueur qui s'étend depuis la partie de bord latéral de la taille jusqu'à la partie de bord latéral de la partie d'entrejambe ; et
chaque organe de protection de fuite est divisé en une zone supérieure (151) et en une zone inférieure (152) au moyen d'une ouverture (153) formée dans une position prédéterminée de la région qui s'étend depuis son bord supérieur jusqu'à son bord inférieur ;
grâce à quoi l'ouverture de chaque organe de protection de fuite fonctionne comme un trou (L) de jambe lorsqu'on porte cet article, la jambe de l'utilisateur étant introduite dans le trou de jambe, la zone supérieure est développée vers l'extérieur de façon à venir en contact avec la partie de taille de l'utilisateur, et la zone inférieure est développée vers l'intérieur de façon à venir en contact avec le côté intérieur de la jambe de l'utilisateur.

26. Article absorbant dans lequel une partie avant (1A) et une partie arrière (1B) sont formées séparément et leurs parties de bord latéral et la partie d'entrejambe sont reliées entre elles, **caractérisé en ce que** l'article comprend :
des organes de protection de fuite (150) qui réunissent l'avant à la partie arrière de l'article absorbant et dont chacun est divisé en une zone supérieure (151) et en une zone inférieure (152), en étant replié vers l'intérieur par rapport à la partie centrale du corps principal dans sa direction de largeur, grâce à quoi la partie avant et la partie arrière sont repliées de façon à se faire face l'une l'autre ;
grâce à quoi, lorsque l'article est porté, la zone supérieure est développée vers l'extérieur de façon à venir en contact avec la partie de taille de l'utilisateur, et la zone inférieure est développée vers l'intérieur de façon à venir en contact avec le côté intérieur de la jambe de l'utilisateur.

27. Article absorbant selon la revendication 25 ou la revendication 26, dans lequel l'organe de protection de fuite comprend un matériau en feuille sans couture d'une structure unique.

28. Article absorbant selon l'une quelconque des revendications 25 à 27, dans lequel l'ouverture est une fente qui s'étend dans la direction de la largeur de l'organe de protection de fuite.

29. Article absorbant selon l'une quelconque des revendications 25 à 28, dans lequel l'ouverture prend la forme d'un triangle sensiblement équilatéral avec une ligne droite qui s'étend dans la direction de la largeur prise comme son côté de base.

30. Article absorbant selon l'une quelconque des revendications 25 à 29, dans lequel la zone inférieure de l'organe de protection de fuite est reliée de façon mutuelle à la partie avant et à la partie arrière au niveau de la partie de bord inférieur, où elle est pliée vers l'intérieur au niveau de la partie centrale lorsqu'on regarde dans la direction de la largeur empêchant ainsi la déformation vers l'extérieur de la zone inférieure.

31. Procédé de fabrication d'un article absorbant comprenant des parties avant et arrière (1A et 1B), reliées entre elles en des parties de bord latéral et en des parties d'entrejambe de ces parties, des organes de protection de fuite (150) fabriqués en un matériau en feuille élastique reliant entre elles les parties de bord latéral qui se font face des parties avant et arrière sur toute la longueur qui s'étend depuis la partie de bord latéral de la taille jusqu'à la partie de bord latéral de la partie d'entrejambe, chaque organe de protection de fuite étant divisé en une zone supérieure (151) et en une zone inférieure (152) au moyen d'une ouverture de jambe (153) formée en une position prédéterminée d'une région qui s'étend depuis son bord supérieur jusqu'à son bord inférieur, le procédé comprenant les étapes consistant à :
superposer des feuilles de dessus et arrière de type bande continue (A1 et A2) de manière à préparer un premier ensemble formé avec un organe absorbant (A3) entre elles avec un intervalle convenable, le premier ensemble servant de partie avant ;
superposer des feuilles de dessus et arrière de type bande continue (B1 et B2) de manière à préparer un second ensemble formé avec un organe absorbant (B3) pris en sandwich entre elles avec un intervalle convenable, le second ensemble servant de partie arrière ;
fournir de façon continue le premier et le second ensembles en un point prédéterminé (P1) de leur trajet de transfert à une vitesse synchronisée de façon qu'ils soient superposés à l'instant où chaque organe absorbant se fait face ;
plier un matériau en feuille continue (C), qui sert d'organe de protection de fuite, en deux au niveau de la partie centrale lorsqu'on regarde dans la direction de sa largeur et en formant l'ouverture (C1) à un intervalle prédéterminé de façon à former un organe susceptible de former un organe de protection de fuite ;
fournir de façon continue un sous-ensemble entre le premier et le second ensembles, plié en deux en un point (P2) qui se trouve au devant du point (P1);
relier le premier et le second ensembles et l'organe susceptible de former un organe de protection de fuite en une partie de liaison prédéterminée ; et
découper le premier et le second ensembles le long d'une ligne de découpe prédéterminée de façon à séparer les articles absorbants individuels.
